# EUROPEAN PATENT APPLICATION

(11) **EP 2 551 353 A1**
(43) Date of publication of application: **30.01.2013**
(21) Application number: 11175242.4
(22) Date of filing: 25.07.2011
(51) Int. Cl.: C12Q 1/68, C12Q 1/70

(54) **Means and methods for predicting the risk of mortality of patients with HPV positive oropharyngeal squamous cell cancer**

(71) Applicant: Deutsches Krebsforschungszentrum, 69120 Heidelberg (DE); Ruprecht-Karls-Universität Heidelberg, 69117 Heidelberg (DE)
(72) Inventor: Schmitt, Markus, 69121 Heidelberg (DE); Pawlita, Michael, 74927 Eschelbronn (DE); Holzinger, Dana, 68167 Mannheim (DE); Bosch, Franz, 69245 Bammental (DE)
(74) Representative: Dick, Alexander

(57) **Abstract**

The present invention relates to the field of diagnostic measures. In particular, the present invention relates to a method for predicting the risk of mortality in a subject suffering from low viral load HPV (human papillomavirus) positive oropharyngeal squamous cell cancer. The method is based on the determination of the amount of an E6* gene product and the amount an E1^E4 gene product of HPV in a sample from said subject. Moreover, the method is based on determining the presence of absence of an E1C gene product of HPV. The present invention further relates to a method for predicting the risk of mortality in a subject suffering from HPV (human papillomavirus) positive oropharyngeal squamous cell cancer based on the determination of copy number of HPV per cancer cell.

## Description

The present invention relates to the field of diagnostic measures. In particular, the present invention relates to a method for predicting the risk of mortality in a subject suffering from low viral load HPV (human papillomavirus) positive oropharyngeal squamous cell cancer. The method is based on the determination of the amount of an E6* gene product and the amount of an E1^E4 gene product of HPV in a sample from said subject. Moreover, the method is based on determining the presence or absence of an E1C gene product of HPV. The present invention further relates to a method for predicting the risk of mortality in a subject suffering from HPV (human papillomavirus) positive oropharyngeal squamous cell cancer based on the determination of copy number of HPV per cancer cell.

First indications for an involvement of human papillomavirus (HPV) in the etiology of head and neck squamous cell carcinomas (HNSCC) have been obtained in the early 1980s (Syrjanen, K., Syrjanen, S., and Pyrhonen, S., Human papilloma virus (HPV) antigens in lesions of laryngeal squamous cell carcinomas. ORL J Otorhinolaryngol Relat Spec 44 (6), 323 (1982)). Subsequently, a specific association of HPV with tumors in Waldeyer's tonsillar ring was found (Andl, T. et al., Etiological involvement of oncogenic human papillomavirus in tonsillar squamous cell carcinomas lacking retinoblastoma cell cycle control. Cancer Res 58 (1), 5 (1998); Paz, I. B. et al., Human papillomavirus (HPV) in head and neck cancer. An association of HPV 16 with squamous cell carcinoma of Waldeyer's tonsillar ring. Cancer 79 (3), 595 (1997); Snijders, P. J. et al., Prevalence and expression of human papillomavirus in tonsillar carcinomas, indicating a possible viral etiology. Int J Cancer 51 (6), 845 (1992)). Andl et al. demonstrated for the first time a longer overall and progression-free survival of patients with HPV-positive tonsillar cancers despite poor tumor differentiation and advanced clinical stages. Since 2000, several larger studies (n>90) confirmed an association between HPV, especially HPV16, and oropharyngeal squamous cell carcinomas (OPSCC) and revealed some distinct differences between HPV-negative and HPV-positive OPSCC regarding gender, age, tumor histology, size and lymph node involvement ((Ang, K. K. et al., Human papillomavirus and survival of patients with oropharyngeal cancer. N Engl J Med 363 (1), 24 (2010); Fakhry, C. et al., Improved survival of patients with human papillomavirus-positive head and neck squamous cell carcinoma in a prospective clinical trial. J Natl Cancer Inst 100 (4), 261 (2008); Gillison, M. L. et al., Evidence for a causal association between human papillomavirus and a subset of head and neck cancers. J Natl Cancer Inst 92 (9), 709 (2000); Klussmann, J. P. et al., Human papillomavirus-positive tonsillar carcinomas: a different tumor entity? Med Microbiol Immunol 192 (3), 129 (2003); Klussmann, J. P. et al., Prevalence, distribution, and viral load of human papillomavirus 16 DNA in tonsillar carcinomas. Cancer 92 (11), 2875 (2001); Lindel, K. et al., Human papillomavirus positive squamous cell carcinoma of the oropharynx: a radiosensitive subgroup of head and neck carcinoma. Cancer 92 (4), 805 (2001); Ritchie, J. M. et al., Human papillomavirus infection as a prognostic factor in carcinomas of the oral cavity and oropharynx. Int J Cancer 104 (3), 336 (2003)). Patients with HPV-positive compared to patients with HPV-negative OPSCC consumed less tobacco and alcohol and had higher numbers of lifetime sex partners and of oral-genital or oral-anal contacts ((Gillison, M. L. et al., Distinct risk factor profiles for human papillomavirus type 16-positive and human papillomavirus type 16-negative head and neck cancers. J Natl Cancer Inst 100 (6), 407; (2008); Hafkamp, H. C. et al., Marked differences in survival rate between smokers and nonsmokers with HPV 16-associated tonsillar carcinomas. Int J Cancer 122 (12), 2656 (2008); Shi, W. et al., Comparative prognostic value of HPV16 E6 mRNA compared with in situ hybridization for human oropharyngeal squamous carcinoma. J Clin Oncol 27 (36), 6213 (2009); Sturgis, E. M. and Cinciripini, P. M., Trends in head and neck cancer incidence in relation to smoking prevalence: an emerging epidemic of human papillomavirus-associated cancers? Cancer 110 (7), 1429 (2007), D'Souza, G. et al., Oral sexual behaviors associated with prevalent oral human papillomavirus infection. J Infect Dis 199 (9), 1263 (2009); Smith, E. M. et al., Age, sexual behavior and human papillomavirus infection in oral cavity and oropharyngeal cancers. Int J Cancer 108 (5), 766 (2004)). Of interest, patients with HPV DNA-positive OPSCC had an improved survival (e.g. D'Souza et al.). However, heterogeneities among HPV-positive OPSCC were also noted and were attributed to differences in viral load and/or viral oncogene expression (see Paz et al., Andl et al., Klussmann et al. 2003, Mellin et al., Wiest et al., Attner et al., Braakhuis et al., Jung et al. ; Schmitt, M. and Pawlita, M., The HPV transcriptome in HPV16 positive cell lines. Molecular and cellular probes 25 (2-3), 108; Schmitt, M. et al., Diagnosing cervical cancer and high-grade precursors by HPV16 transcription patterns. Cancer Res 70 (1), 249 (2010)) Both the analysis of viral load and of viral oncogene expression have been applied to identify HNSCC with biologically active HPV16 (Braakhuis, B. J. et al., Genetic patterns in head and neck cancers that contain or lack transcriptionally active human papillomavirus. J Natl Cancer Inst 96 (13), 998 (2004), Jung et al. , Klussmann et al. 2001, Lindquist, D. et al., Human papillomavirus is a favourable prognostic factor in tonsillar cancer and its oncogenic role is supported by the expression of E6 and E7. Mol Oncol 1 (3), 350 (2007), Wiest, T. et al., Involvement of intact HPV16 E6/E7 gene expression in head and neck cancers with unaltered p53 status and perturbed pRb cell cycle control. Oncogene 21 (10), 1510 (2002)).

In cervical carcinogenesis the oncogenic potential of high-risk HPV is mediated through continuous expression of the two major viral oncoproteins E6 and E7 18. Integration of the HPV genome into the tumor cell genome has been proposed to strongly enhance the expression of E6 and E7 due to the disruption of the open reading frame of the E2 transcriptional repressor (ref. Munger 1989; Schwarz 1985 Schwarz E, Freese UK, Gissmann L, Mayer W, Roggenbuck B, Stremlau A, et al. Structure and transcription of human papillomavirus sequences in cervical carcinoma cells. Nature. 1985;314:111-4, Munger K, Phelps WC, Bubb V, Howley PM, Schlegel R. The E6 and E7 genes of the human papillomavirus type 16 together are necessary and sufficient for transformation of primary human keratinocytes. J Virol. 1989;63:4417-21). An HPV16 RNA pattern consistent with integration would show upregulated E6*II (226^526) and/or E6*I (226^409) transcripts with a concurrently low or absent expression of the E1^E4 (880^3358) transcript (herein referred to as pattern 1), (Schmitt, M. et al., Diagnosing cervical cancer and high-grade precursors by HPV16 transcription patterns. Cancer Res 70 (1), 249 (2010). This pattern was found in many but not all of HPV16-positive cervical carcinomas (CxCa) and cervical high-grade intraepithelial lesions (HSIL). In CxCa and HSIL lacking pattern 1, another potentially diagnostic pattern was found: up-regulated E1C (880^2582) transcript with a concurrently low or absent L1 (3632^5639) transcript (herein referred to as pattern 2). The function of E1C remains unclear (Schmitt, M. and Pawlita, M., The HPV transcriptome in HPV16 positive cell lines. Molecular and cellular probes 25 (2-3), 108)).

Compared to CxCa, in HPV-positive OPSCC a lower rate (0-41%) of HPV DNA integration has been reported (Hafkamp et al.; Wiest et al.; Mellin, H. et al., Human papillomavirus type 16 is episomal and a high viral load may be correlated to better prognosis in tonsillar cancer. Int J Cancer 102 (2), 152 (2002)). Whether they express viral RNA patterns as in CxCa is unclear, since the analyses of viral transcription were confined to full-length E6 and E7 RNA (Wiest, T. et al., Attner, P. et al., The role of human papillomavirus in the increased incidence of base of tongue cancer. Int J Cancer 126 (12), 2879 (2010); Lindquist, D. et al., Human papillomavirus is a favourable prognostic factor in tonsillar cancer and its oncogenic role is supported by the expression of E6 and E7. Mol Oncol 1 (3), 350 (2007); Braakhuis, B. J. et al., Genetic patterns in head and neck cancers that contain or lack transcriptionally active human papillomavirus. J Natl Cancer Inst 96 (13), 998 (2004))

Thus, there is still a strong need for more reliable methods for predicting the risk of mortality in a subject suffering from HPV positive oropharyngeal squamous cell cancer (OPSCC).

Jung et al. (Jung, et al., Int J Cancer 126 (8), 1882 (2010)) analyzed HPV viral load and transcription in 231 patients with head and neck squamous cell carcinoma. HPV transcription was assessed by determining the presence of E6/E7 mRNAs levels. Twelve out of 30 HPV16 DNA-positive tumors displayed high E6/E7 mRNAs levels. While HPV-free and non-transcriptionally active HPV-related patients showed similar 5-years survival rates, E6/E7 expression was associated with a better prognosis. Moreover, Jung correlates viral load values to the presence of transcripts. Jung concludes that the tumors in which the HPV16 was transcriptionally active had elevated viral load values and a better prognosis than patients without detectable transcripts. However, patients with low viral loads had similar survival rates and transcriptional profiles as HPV negative patients. Jung, therefore, concludes that it "would be reasonable to consider the cases with low HPV DNA load to be HPV negative".

In contrast, it was found in the studies carried out in the context of the present invention that some patients, although having a low viral load, may have a better prognosis than HPV negative patients. In particular, the risk of mortality is decreased if the patients with low viral load show a certain HPV transcriptional pattern as described herein below. Moreover, it was shown that not all transcriptionally active patients have a good prognosis. Rather, it was shown that patients had the best prognosis, if they showed a specific RNA expression pattern. Accordingly, the present invention relates to a method for predicting the risk of mortality in a subject suffering from HPV positive oropharyngeal squamous cell cancer (OPSCC), in particular from low viral load HPV positive OPSCC, comprising the steps of:
a) determining the amount of an E6* gene product and the amount of an E1^E4 gene product of HPV in a sample from said subject, calculating a ratio of the amount of the E6* gene product to the amount of the E1^E4 gene product, and comparing the, thus, calculated ratio to a reference ratio of the amount of the E6* gene product to the amount of the E1^E4 gene product, and
b) asssessing the presence or absence of an E1C gene product in a sample from said subj ect,
whereby the risk of mortality in said subject is to be predicted.

Preferably, it is predicted whether the subject is of risk of mortality, or not, by carrying out the further step of c) predicting the risk of mortality, or not, based on the result of the comparison carried out in step a) and based on the assessment carried out in step b).

The method of present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination or a computer-implemented comparison based on said comparison. More preferably, the method is carried out entirely in an automated manner. In such a case, the diagnostic result which is established in step b) (or c)) is generated in a suitable output format so that it can be used as an aid for establishing the final clinical diagnosis by, e.g., a medical practitioner.

In the context of the method of the present invention, the risk of mortality of a subject shall be predicted. The term "predicting the risk" as used herein, preferably, refers to assessing the probability according to which the subject as referred to herein will die. More preferably, the risk/probability of mortality within a certain time window is predicted. In a preferred embodiment of the present invention, the predictive window, preferably, is an interval of at least 6 months, at least 9 months, at least 1 year, at least 2 years, at least 3 years, at least 4 years, at least 5 years, at least 10 years, at least 15 years or any intermitting time range. In a particular preferred embodiment of the present invention, the predictive window, preferably, is an interval of 3 years, or more preferably, of 5 years. Preferably, said predictive window is calculated from the time point at which the sample to be tested has been obtained.

As will be understood by those skilled in the art, the aforementioned prediction is usually not intended to be correct for 100% of the subjects to be analyzed. The term, however, requires that the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.001. Preferably, the probability envisaged by the present invention allows that the differentiation will be correct for at least 60%, at least 70%, at least 80%, or at least 90% of the subjects of a given cohort. Preferably, the probability envisaged by the present invention allows that the prediction of an increased, normal or decreased risk will be correct for at least 60%, at least 70%, at least 80%, or at least 90% of the subjects of a given cohort or population. The term "predicting", preferably, relates to predicting whether a subject is at elevated risk or reduced risk as compared to the average risk in a population of subjects with HPV positive OPSCC. Of course, the risk of mortality due to, i.e. caused by, OPSCC shall be predicted.

The term "predicting the risk of mortality" as used herein means that the subject to be analyzed by the method of the present invention is allocated either into the group of subjects being at risk of mortality, or into a group of subjects being not at risk of mortality. Having a risk of mortality as referred to in accordance with the present invention, preferably, means that the risk of mortality is increased (preferably, within the predictive window). Preferably, said risk is elevated as compared to the average risk in a cohort of subjects suffering from HPV positive OPSCC. A subject who is not at risk of mortality as referred to in accordance with the present invention, preferably, has a reduced risk of mortality (within the predictive window). Preferably, said risk is reduced as compared to the average risk in a cohort of subjects HPV positive OPSCC. A subject who is at risk of mortality preferably has a risk of 50% or larger, or, more preferably of 60 % or larger of mortality, preferably, within a predictive window of 5 years. A subject who is not at risk of mortality, preferably, has a risk of mortality of lower than 40 %, and more preferably of lower than, 30 % or lower preferably, within a predictive window of 5 years.

The term "subject" as used herein relates to animals, preferably mammals, and, more preferably, humans. The subject to be tested in the context of the method of the present invention shall suffer from oropharyngeal squamous cell cancer (abbreviation: OPSCC), in particular from HPV positive OPSCC.

Oropharyngeal squamous cell cancer is a type of head and neck cancer that occurs in the oropharynx, the middle part of the throat that includes the soft palate, the base of the tongue, the tonsils and the side and back wall of the throat. Squamous cell cancers of the tonsils are more strongly associated with HPV (Human papillomavirus) infection than are cancers of other regions of the head and neck. HPV-positive oropharyngeal squamous cell cancer is a subtype of oropharyngeal squamous cell cancer associated in >95% with the HPV type 16. Thus, the subject shall, preferably, suffer from HPV16 positive OPSCC. HPV16 belongs to the high-risk HPV genotypes and is the main cause for the development of cervical cancer. HPV oral infection precedes the development of HPV positive OPSCC. Slight injuries in the mucous membrane serve as an entry gate for HPV, which thus works into the basal layer of the epithelium.

The human papillomavirus is a DNA virus that infects the skin and mucous membranes. More than 100 HPV genotypes have been described (de Villiers, E. M., C. Fauquet, T. R. Broker, H. U. Bernard, and H. zur Hausen. 2004. Classification ofpapillomaviruses. Virology 324:17-27). It is also known that HPV can cause vulvar, anal, vaginal, penile and oropharyngeal cancer, as well as vaginal intraepithelial neoplasia, anal intraepithelial neoplasia, and vulvar intraepithelial neoplasia. The term "HPV", as used herein, preferably, relates to HPV16.

Preferably, the subject to be tested in the context of the method of the present invention suffers from low viral load HPV positive OPSCC. A subject, preferably, suffers from low viral load HPV positive OPSCC, if the subject comprises less than 10 copies of the HPV16 genome per cancer cell, in particular per OPSCC cancer cell. More preferably, the subject comprises less than 5 copies of the HPV genome per cancer cell, and, most preferably, less than 1 copy of the HPV genome per cancer cell, in particular per OPSCC cancer cell. It is further preferred that the subject suffering from low viral load HPV comprises less than 0.5 copies or less than 0.3 of the HPV genome per cancer cell. Also preferred is that the subject suffering from low viral load HPV comprises less than 0.1 copies or less than 0.05 copies of the HPV16 genome per cancer cell. How to determine the copy number of HPV per cancer cell is well known in the art. E.g. it can be determined by the methods described in the Examples. The indicated copy numbers preferably, relate to the average copy number of the HPV genome in the (OPSCC) cancer cells comprised by the entire tumor or by the sample to be tested. The term "average" as used herein, preferably, means the arithmetical average.

Preferably, the copy number of the HPV genomes encompasses both the HPV genomes present in an integrated (i.e. integrated into chromosomal DNA) and episomal form in the cells.

The person skilled in the art is aware that not the complete HPV genome (i.e. the entire sequence of the HPV genome) is integrated into the chromosomal DNA if the HPV genome is present in an integrated form. Rather, in the majority of cases, only the E6, E7, E1, and L1 polynucleotides are integrated. Accordingly, the "copy number of the HPV" genome as used herein, preferably, is intended to relate to the number of copies of the E1 polynucleotide, L1 polynucleotide, or more preferably, the E6 polynucleotide or E7 polynucleotide present in the cell (i.e. to average number of copies). The sequence of the E6 polynucleotide is shown in SEQ ID NO: 30, the sequence of the E7 polynucleotide is shown in SEQ ID NO: 31.

In an embodiment of the present invention, the aforementioned method comprises the step of determining the copy number of HPV per cancer cell in a sample from said subject. Preferably, said step is carried out before step a) of the aforementioned method. Preferably, the further steps a) and b) are only carried out in subjects which have a low viral load of HPV (as determined by the step of determining the copy number of HPV per cancer cell in a sample from said subject)

A sample can be obtained by well-known techniques and include samples from those cells, tissues or organs which express or produce the gene products referred to herein. Preferably, the sample comprises OPSCC tumor tissue. Preferably, the sample is a scrape, and, more preferably, a biopsy from the oropharyngeal tract. Thus, the sample to be tested is, in particular, a biopsy of a squamous cell carcinoma of the oropharynx. It is also envisaged that the sample is obtained during the removal of the tumor by surgery. Preferably, the sample to be tested in the context of the present invention comprise OPSCC cells (and, thus, tumor cells). In particular, is it envisaged that at least 25%, or, more preferably, at least 50 % cells comprised by the samples are OPSCC cells.

Separated cells may be obtained from the body fluids or the tissues or organs by separating techniques such as filtration, centrifugation or cell sorting. Moreover, the sample may be further processed by well-known methods in order to further enrich and/or purify the gene products as referred to herein. The further processing of a gene product, preferably, depends on the nature of the gene product, i.e. whether the gene product is a polypeptide or an RNA molecule. Preferably, if the gene product is a polypeptide, then polypeptides are enriched and/or purified by methods well known by the skilled person. Preferably, if the gene product is an mRNA molecule, then said RNA molecules may enriched and/or purified by methods well known in the art.

The term "gene product" as used herein, preferably, refers to a transcript, and thus to mRNA, or to a polypeptide. In particular, it is envisaged that the gene products are spliced mRNAs/transcripts of HPV16. Throughout the specification, the term "spliced mRNA" and "spliced transcript" are used interchangeably.

The HPV 16 genome consists of a single molecule of double-stranded, circular closed DNA with approximately 7,906 base pairs (bp). The nucleic acid sequence of the HPV16 genome, HPV16R, is shown in SEQ ID NO: 1 (see. e.g. Myers, G., H. Delius, J. Icenogle, H. U. Bernard, M. Favre, M. van Ranst, and C. M. Wheeler. 1997. Human papillomaviruses 1997: a compilation and analysis of nucleic acid and amino acid sequences. Theoretical Biology and Biophysics, Los Alamos National Laboratory, Los Alamos, N.Mex, see also SEQ ID NO: 1 of EP 2 184 368 A1). Three open reading frames (ORF) are located on one strand. Three functional areas have been defined, the long control region (LCR), and the "early" and the "late" transcription regions. The LCR is an 850 bp long non-coding upstream region responsible for the regulation of DNA replication and transcription. It contains several binding sites for the viral E2 and other cellular transcription factors and a binding site for the viral E1 replication protein. Furthermore, it contains silencer as well as enhancer sequences and harbours the p97 core promoter close to the E6 ORF; it is the region of the highest degree of variation in the viral genome. The "early" region, consists of the ORF E1, E2, E4, E5, E6 and E7, which are involved in viral replication and cell transformation. The "late" region encodes the L1 and L2 structural proteins that form the viral capsid. Of the "early" proteins, the two most important HPV proteins for malignant diseases are E6 and E7, which act synergistically to transform cells from normal to immortal state. It is known in the art that the HPV16 transcriptom exhibits several splice donor (at nucleotide positions 226, 880, 1302 and 3632 of the HPV16R reference genome) and splice acceptor sites (at nucleotide positions 409, 526, 742, 2582, 2709, 3358 and 5639 of the HPV16R reference genome) resulting in at least 11 different splice junctions (Baker, C., and C. Calef. 1996. Maps of papillomavirus mRNA transcripts. Los Alamos National Laboratories, Los Alamos, NM, USA.; Zheng, Z. M., and C. C. Baker. 2006. Papillomavirus genome structure, expression, and post-transcriptional regulation. Front Biosci 11:2286-302.). Splicing products are characterized herein based on the splice donor and acceptor sites used for generating the products. The respective splice donor and acceptor are separated by "^". The spliced mRNAs preferably comprise the respective splice junction. The sequence of the splice junctions are well known in the art, and e.g. disclosed in EP 2 184 368 A1, in particular in table 7 (which is herewith incorporated by reference).

The gene products to be determined in the context of the present invention are well known in the art. E.g. they are also disclosed in EP 2 184 368 A1 which herewith is incorporated by reference with respect to its entire disclosure content (in particular with respect to the gene products and the sequences of the gene products mentioned in this specification and with respect to methods for the determination of these gene products, in particular with respect to the sequences of probe oligonucleotides and the sequences of the oligonucleotides for the amplification of gene products).

In step a) of the aforementioned method, the amounts of an E6* gene product of HPV16 and of an E1^E4 gene product shall be determined in the sample.

In a preferred embodiment of the method of the present invention, the E6* gene product is an E6*I or E6*II gene product. It is, thus, envisaged to determine the amount of the E6*I or E6*II gene product, or of both the E6*I and E6*II gene product. Most preferred is the determination of an E6*I gene product.

The term "E6*I gene product" as used herein, preferably, refers to 226^409 spliced mRNAs of HPV16 or to the E6*I polypeptide of HPV encoded by said 226^409 spliced mRNA. It has been suggested that E6*I polypeptide may transactivate the virus LCR (Alloul, N., and L. Sherman. 1999. Transcription-modulatory activities of differentially spliced cDNAs encoding the E2 protein of human papillomavirus type 16. J Gen Virol 80 (Pt 9):2461-70.). The amino acid sequence of the E6*I polypeptide is shown in SEQ ID NO: 2. The nucleic acid sequence encoding said polypeptide is shown in SEQ ID NO: 3.

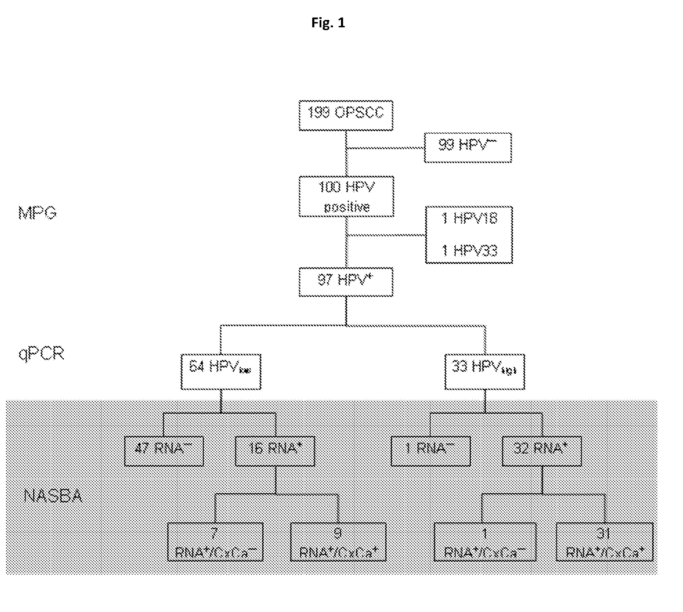

In particular, the E6*I gene product is a 226^409 spliced mRNA of HPV16. 226^409 spliced mRNAs, preferably, comprise the nucleic acid sequence that is generated by linking the 226 donor nucleotide to the 409 acceptor nucleotide. Accordingly, 226^409 spliced mRNAs, preferably, comprise the said nucleic acid sequence. Preferably, said spliced mRNAs comprise a nucleic acid sequence as shown in SEQ ID NO: 4 (cgtgaggtgtat, indicated in the sequence listing is the DNA sequence, in the RNA transcript the T is replaced by U). Accordingly, the E6*I polypeptide of HPV16 preferably comprises an amino acid sequence as shown in SEQ ID NO: 5 (Leu Leu Arg Arg Glu Val Tyr). It is known in the art that various mRNA species of HPV16 comprise 226^409 spliced sequences (in particular, mRNA species B, G and L as shown in Fig. 1 of EP 2 184 368 A1 comprise the 226^409 splice junction. Accordingly, the determination of the amount of 226^409 spliced transcripts, preferably, encompasses the determination the cumulative amount of all mRNA species comprising the 226^409 splice junction.

The term "E6*II gene product" as used herein, preferably, refers to 226^526 spliced mRNAs of HPV16 or the E6*II polypeptide of HPV16 encoded by said 226^526 spliced mRNAs. The amino acid sequence of the E6*II polypeptide is shown in SEQ ID NO: 6. The nucleic acid sequence encoding said E6*II polypeptide is shown in SEQ ID NO: 7.

In particular, the term refers to 226^526 spliced mRNAs. Preferably, 226^526 spliced transcripts are HPV mRNAs that comprise the 226^526 splice junction. Thus, said mRNAs are mRNAs encoded by HPV16R that are the result of splicing the HPV16 transcript at nucleic acid position 226 (donor nucleotide) and 526 (acceptor nucleotide) and connecting the donor nucleotide with the acceptor nucleotide. In the context of the present invention, the first number for spliced mRNAs, here 226, preferably, indicates the position of the donor nucleotide for splicing and thus the 5'-splice junction, whereas the second number, here 526, indicates the position of the acceptor nucleotide for splicing, and, thus, the 3'-splice junction. The indicated positions are drawn to the 7906 bp genome of HPV16R as shown in SEQ ID NO: 1. It is known in the art that various mRNA species of HPV16 comprise 226^526 spliced sequences (in particular, mRNA species C, H, M as shown in Fig. 1 of EP 2 184 368 A1 comprise the 226^526 splice junction. Accordingly, the determination of the amount of *226^526* spliced transcripts, preferably, encompasses the determination the cumulative amount of all mRNA species comprising the 226^526 splice junction.

The term "E1^E4 gene product" as used herein, preferably, refers to 880^3358 spliced mRNAs of HPV16, or the polypeptide of HPV encoded by said 880^3358 spliced mRNA, said polypeptide preferably being a fusion polypeptide of the N-terminus of the E1 polypeptide with the E4 polypeptide of HPV16. Said fusion polypeptide is frequently also referred to as E1^E4. Said polypeptide is expressed in the late phase of the viral life cycle. It is detected in the spinous and granular cell layers and has several functions late in infection of HPV16. The amino acid sequence of the fusion polypeptide is shown in SEQ ID NO: 8. The nucleic acid sequence of encoding said fusion peptide is shown in SEQ ID NO: 9.

880^3358 spliced mRNAs, preferably, comprise the nucleic acid sequence that is generated by linking the 880 donor nucleotide to the 3358 acceptor nucleotide. Accordingly, 880^3358 spliced mRNAs, preferably, comprise the said nucleic acid sequence with the 880^3358 splice junction. Preferably, said spliced mRNAs comprise a nucleic acid sequence as shown in SEQ ID NO: 14 (The "R" in SEQ ID NO: 14 represents A or G, two naturally occurring variants). Accordingly, the E1^E4 fusion polypeptide of HPV16 preferably comprises an amino acid sequence as shown in SEQ ID NO: 16. Several spliced transcripts comprise the 880^3358 splice junction, and, thus encode the gene product of E1^E4 in particular species A-D, and Q-S as shown in Fig. 1 of EP 2 184 368 A1.

The gene product mentioned in step b) in the context of the aforementioned method of the present invention is the E1C gene product. Preferably, the E1C gene product is the E1C polypeptide. More preferably, the E1C gene product is a 880^2582 spliced transcript. 880^2582 spliced transcripts are alternatively spliced transcripts of HPV16R spliced at position 880 and 2582 (see below). In particular, said 880^2582 spliced transcripts shall encode for the E1C polypeptide.

The E1C polypeptide is an N-terminally truncated variant of the E1 polypeptide of HPV. It has been proposed to act as a trans-activator of LCR. The amino acid sequence of E1C of HPV16R is shown in SEQ ID NO: 10. The nucleic acid sequence encoding said E1C polypeptide of HPV16R is shown in SEQ ID NO: 11 (880^2582 spliced transcript)

Preferably, 880^2582 spliced transcripts are HPV mRNAs that comprise the 880^2582 splice junction. Thus, said mRNAs are mRNAs encoded by HPV16R that are the result of splicing the HPV16 transcript at nucleic acid position 880 (donor nucleotide) and 2582 (acceptor nucleotide) and connecting the donor nucleotide with the acceptor nucleotide. In the context of the present invention, the first number for spliced mRNAs, here 880, preferably, indicates the position of the donor nucleotide for splicing and thus the 5'-splice junction, whereas the second number, here 2582, indicates the position of the acceptor nucleotide for splicing, and, thus, the 3'-splice junction. The indicated positions are drawn to the 7906 bp genome of HPV16R as shown in SEQ ID NO: 1. It is known in the art that various mRNA species of HPV comprise 880^2582 spliced sequences (see e.g. species K-N in Fig. 1 of EP 2 184 368 A1) Accordingly, the determination of the amount of 880^2582 spliced transcripts, preferably, encompasses the determination the cumulative amount of all mRNA species comprising the 880^2582 splice junction. Thus, the 880^2582 spliced transcript shall be generated by linking the 880 donor nucleotide to the 2582 acceptor nucleotide. Thus, the said transcript shall comprise the splice junction after splicing. Accordingly, 880^2582 spliced transcripts, preferably, comprise the said splice junction generated by splicing. Preferably, said spliced transcripts comprise a nucleic acid sequence as shown in SEQ ID NO: 12 The "Y" in SEQ ID NO: 12 represents two naturally occurring variants.

Preferably, the E1C polypeptide is translated from/encoded by the polynucleotide that comprises the aforementioned splice junction. Accordingly, the E1C polypeptide of HPV16 preferably comprises an amino acid sequence as shown in SEQ ID NO: 13.

Thus, particular preferred gene products are as follows: Preferred E1C gene products are spliced transcripts comprising the 880^2582 junction. Preferred E6* gene products are spliced transcripts comprising the 226^409 junction and/or spliced transcripts the 226^526 junction. Preferred E1^E4 gene products are spliced transcripts comprising the 880^3358 junction.

The determination of the amount of a gene product, preferably, depends on the nature of the gene product, i.e. whether the gene product is a transcript or a polypeptide.

Determining the amount of a transcript, and thus the amount of a mRNA, in a sample of a subject can be done by any method deemed appropriate.

Preferably, the amount of a transcript is determined by using a probe oligonucleotide that specifically detects the transcript to be analyzed.

The determination of the amount of a transcript or an amplification product thereof, by specific probe oligonucleotides, preferably, comprises the step of hybridizing a transcript or an amplification product (for an explanation of "amplification products", see below) thereof with probe oligonucleotides that specifically bind to the transcript or the amplification product thereof. A probe oligonucleotide in the context of the present invention, preferably, is a single-stranded nucleic acid molecule that is specific for said transcript or the amplification product thereof. The skilled person knows that a probe oligonucleotide comprises a stretch of nucleotides that specifically hybridizes with the target and, thus, is complementary to the target polynucleotide. Said stretch of nucleotides is, preferably, 85%, 90%, 95%, 99% or more preferably 100% identical to a sequence region comprised by a target polynucleotide.

In order to allow specific detection of a transcript or amplification product thereof, the probe oligonucleotide, preferably, specifically binds to the transcript or amplification product to be detected, but not to other polynucleotide comprised by said sample. How to choose suitable probe oligonucleotides is known in the art.

The probe oligonucleotides of the present invention may be labelled or contain other modifications including enzymes which allow a determination of the amount of a transcript or an amplification product thereof. Labelling can be done by various techniques well known in the art and depending of the label to be used. Preferred labels are described elsewhere in this specification.

The probe oligonucleotide may be bound to a solid surface or present in a liquid phase. As an example, the probe oligonucleotides are bound to a carrier providing a solid surface. Preferably, said carrier is a small particle or bead. The overall size of a small particle or bead, preferably, may be in the micrometer or nanometer range. Said beads and particles may be stained with a specific dye, more preferably with a specific fluorescent dye. Preferably, by staining various carriers with various dyes, the carries can be distinguished from each other. By using a carrier with a specific dye for a specific probe oligonucleotide (thus, a nucleic acid that targets the amplified polynucleotides of a specific sequence), said carrier is distinguishable from other carriers comprising different dyes. In one preferred embodiment commercially available Luminex microspheres (Luminex Corp., Austin, Texas, USA) are used. Thus, for detection of a transcript or amplification product thereof, the probes are coupled to fluorescence-labelled polystyrene beads (Luminex suspension array technology) which are hybridized with the amplification products under suitable, preferably, stringent conditions. Moreover, the amplification products may be identified by use of microarrays, Reverse-Line Blots (RLB), Dot blots or similar technologies which contain specific oligonucleotides linked to a suitable carrier. Probe oligonucleotides present in a liquid phase may bind to immobilised target nucleic acid molecules or amplified polynucleotides. Specific labels or modifications known by persons skilled in the art may allow target detection or signal amplification. In addition, amplification products may be detected by size separation e.g. gel or capillary electrophoresis, by nucleotide composition, using e.g. Nuclear Magnetic Resonance, or by real-time PCR and signal amplification methods as described elsewhere herein.

The person skilled in the art is able to select suitable probe oligonucleotides. For the determination of spliced transcripts, it is particularly contemplated to determine the amount of said alternatively spliced mRNAs by using probe oligonucleotides that specifically bind to the splice junction, and, thus bind the nucleic acid sequence that is generated by connecting the respective specific splice donor and splice acceptor nucleotide. Of course, said probe oligonucleotide also binds to the amplification products of the respective transcripts.

A probe oligonucleotide for the determination of 880^2582 spliced mRNAs, preferably, comprises a nucleic acid sequence as shown in SEQ ID NO: 12.

Also, a probe oligonucleotide for the determination of 880^3358 spliced mRNAs, preferably, comprises a nucleic acid sequence as shown in SEQ ID NO: 14.

Moreover, a probe oligonucleotide for the determination of E6*I and/or E6*II transcripts, and thus of 226^409 or 226^526 spliced mRNAs, preferably, comprises a nucleic acid sequence as shown in SEQ ID NO: 15.

Moreover, a probe oligonucleotide for the determination of 3632^5639 spliced mRNAs, preferably, comprises a nucleic acid sequence as shown in SEQ ID NO: 29.

Particularly preferred probe olignonucleotides for the determination of the gene products as mentioned herein are those disclosed in table 1 of EP 2 184 368 A1 (which table is herewith incorporated by reference).

Preferably, the determination of the amount of a transcript comprises the steps of amplifying the said transcript with oligonucleotides that specifically amplify said transcript and determining the amount of the, thus, amplified transcripts. Thus, for determination of the amount of a transcript, it is particularly preferred to amplify the transcript by suitable methods described elsewhere herein, and then to determine the amount of the amplification product. Alternatively, the determination of the amount of a transcript is achieved by signal amplification methods with oligonucleotide probes that specifically bind said transcript and allow linear signal amplification and subsequent determination of the amplified signal.

How to amplify a transcript is well known in the art. Amplification of a transcript, preferably, is a template-dependent process which results in an increase of the amount of a corresponding nucleic acid molecule relative to the initial amounts. The amplification product, preferably, is a nucleic acid, DNA or RNA. It is to be understood that amplification of a transcript may comprise additional steps such as reverse transcription of the transcript by well known methods.

How to amplify a target signal is well known in the art. Amplification of a signal, preferably, is a template-dependent process which results in an increase of the amount of a reporter signal relative to the initial amounts. The reporter signal, preferably, is a visible light, fluorescence, chemiluminescence, and luminescence. Methods for signal amplification are well-known in the art and may be based on tyramide signal amplification, branched DNA amplification, Dendrimer® amplification, padlock probes and rolling circle amplification, Invader® signal amplification and other signal amplification methods.

The amplification of a transcript of interest may be carried out by well-known methods, preferably by polymerase chain reaction (PCR), by reverse transcriptase PCR, real-time PCR, nucleic acid sequence-based amplification (NASBA), transcription-mediated amplification (TMA) and other isothermal amplification methods using polymerases and specific oligonucleotides as primers. PCR methods are well known in the art. Preferably, the amplification is by using suitable oligonucleotides pairs.

The current invention is not restricted to any of the aforementioned technologies. As an exemplary method for the amplification of transcripts, NASBA technology will be briefly summarised. NASBA is an oligonucleotide-dependent technology for the amplification of nucleic acids at one temperature. The sample comprising the transcript to be amplified is added to a reaction mixture comprising at least two transcript specific oligonucleotides for the amplification of said transcript. The first oligonucleotide, containing the T7 RNA promoter sequence, binds to its target site at the 3' end of the template. By reverse transcription a RNA/DNA hybrid is generated. The enzyme RNAse H degrades the RNA portion. After degradation of the RNA template, the second oligonucleotide binds to the 3'-end of the single-stranded cDNA and double-stranded DNA containing an intact T7 RNA promoter is generated. Then, the enzyme T7 RNA polymerase linearly generates antisense RNA. Each newly synthesized antisense RNA molecule can itself act as a template with the second primer and is converted to a DNA intermediate with a functional T7 promoter. However, in this case the oligonucleotide primers anneal in reverse order because the newly generated RNA molecules are opposite in orientation to the original target and the resulting DNA intermediate is only partly double-stranded. In this manner, many RNA copies are generated from each RNA target that re-enter the reaction resulting in the linear synthesis of RNA products under isothermal conditions. An approximately 106- to 109-fold amplification is obtained within 90 min (Compton, J. 1991. Nucleic acid sequence-based amplification. Nature 350:91-2).

In order to specifically amplify spliced mRNAs as referred to herein, the oligonucleotide pair for the amplification of the transcript, preferably, shall be capable to specifically amplify the nucleic acid region that comprises the respective splicing junction. Therefore, the oligonucleotides for the amplification shall specifically bind the transcript (or the complementary strand thereof, particularly a complementary DNA or RNA strand that is generated by approaches described elsewhere herein) 5' and 3' from the splicing junction (one primer 3', one primer 5'). An amplification product generated by using the aforementioned oligonucleotides will comprise the respective splice junction.

Particularly preferred olignonucleotides for the amplification of the gene products as mentioned herein are those disclosed in table 3 of EP 2 184 368 A1 (which table is herewith incorporated by reference).

Accordingly, preferred oligonucleotides for the amplification of 880^2582 spliced mRNAs, preferably, comprise a nucleic acid sequence as shown in SEQ ID NO: 17 and in SEQ ID NO:18.

Preferred oligonucleotides for the amplification of 880^3358 spliced mRNAs, preferably, comprise a nucleic acid sequence as shown in SEQ ID NO: 18 and in SEQ ID NO: 19.

Preferred oligonucleotides for the amplification of E6*I transcripts, and thus of 226^409 spliced mRNAs, preferably, comprise a nucleic acid sequence as shown in SEQ ID NO: 20 and in SEQ ID NO: 21.

Preferred oligonucleotides for the amplification of E6*II transcripts, and thus of 226^526 spliced mRNAs, preferably, comprise a nucleic acid sequence as shown in SEQ ID NO: 22 and in SEQ ID NO: 21.

Preferred oligonucleotides for the amplification of 3632^5639 spliced mRNAs, preferably, comprise a nucleic acid sequence as shown in SEQ ID NO: 32 and in SEQ ID NO:33. Determining the amount of polynucleotides or amplification products referred to in this invention relates to measuring the amount or concentration, preferably semi-quantitatively or quantitatively. Preferably, the determination includes a normalization step for the quantification of transcripts. Exemplarily, this normalization process will be briefly described for NASBA target amplification method. Normalization and thus quantification is preferably achieved by adding a predefined amount of calibrator RNA (Q-RNA) to the amplification mixture. Said calibrator RNA, preferably, shall be in vitro-transcribed RNA that can be amplified by the same oligonucleotides that are capable of specifically amplifying the transcripts to be analyzed. However, said Q-RNAs shall comprise a specific target region for a probe oligonucleotide (i.e. a target region not comprised by the transcript to be analyzed). Said specific target region shall allow for differentiating between the amplification product of the transcript to be analyzed and the amplification product of the Q-RNA. The principle of the normalization is the competitive co-amplification of Q-RNA and the mRNA to be analyzed with the same oligonucleotide pair (van Gemen et al. 1993: Quantification of HIV-1 RNA in plasma using NASBA during HIV-1 primary infection. J Virol Methods 43:177-87). It is to be understood that Q-RNA amounts, preferably, need to be titrated for each mRNA to be analyzed in the context of the present invention. For quantification expression levels can be compared to a standard curve using in vitro transcribed mRNA or to suitable reference material. This can be done by the skilled person without further ado.

An oligonucleotide for the amplification of transcripts in the context of the present invention shall comprise a number of nucleotides being sufficient for specific binding to a sequence stretch of a target polynucleotide. Preferably, an oligonucleotide as meant herein has between 15 and 35 nucleotides in length, more preferably between 18 and 30 nucleotides in length, and most preferably between 20-27 nucleotides in length. A probe oligonucleotide in the context of the present invention allows detection of a transcript as referred to herein and/or amplification products of said transcript (see elsewhere herein). By detecting a transcript or an amplification product thereof, the amount of a specific transcript can be assessed in a sample of a subject with HPV16. In order to allow specific detection of a transcript or an amplification product thereof, the probe oligonucleotide has to be sufficiently complementary to the transcript or amplification product thereof, or to parts of said transcript or said amplification product. Particularly preferred oligonucleotides have the specific sequences and/or properties referred to herein.

Particularly, the oligonucleotides may be biotinylated in order to enable the binding of the amplification products to a streptavidin surface or fluorescent conjugate. Moreover, labels to be used in the context of the present invention may be, but are not limited to, fluorescent labels comprising, inter alia, fluorochromes such as R-phycoerythrin, Cy3, Cy5, fluorescein, rhodamin, Alexa, FAM, HEX, Cy5.5, Coumarin, LC610, LC670 or Texas Red. However, the label may also be an enzyme or an antibody. It is envisaged that an enzyme to be used as a label will generate a detectable signal by reacting with a substrate. Suitable enzymes, substrates and techniques are well known in the art. An antibody to be used as label may specifically recognize a target molecule which can be detected directly (e.g., a target molecule which is itself fluorescent) or indirectly (e.g., a target molecule which generates a detectable signal, such as an enzyme). Moreover, the oligonucleotides may contain generic sequences that allow detection by hybridisation to complementary detector probes that may contain any of the aforementioned labels or modifications. The oligonucleotides of the present invention may also contain 5'-restriction sites, locked nucleic acid molecules (LNA) or be part of a peptide nucleic acid molecule (PNA). Such PNA can be, in principle, detected via the peptide part by, e.g., antibodies.

Also contemplated for the determination of the amount of a transcript (or an amplification product thereof) is the use of array-based techniques for determining the amount of transcripts in accordance with the present invention. An array as referred to herein is a system that comprises a solid support, e.g. a microarray with e.g. a small membrane, a nylon membrane, or glass slide, containing samples of various immobilized polynucleotides arranged in a regular pattern. Alternatively, a bead-array may consist of distinctly fluorescence-labelled microspheres or beads that allow multiplexing of probe oligonucleotides. The oligonucleotides probes, preferably represent genes, i.e. they consist of or comprise a nucleic acid sequence of the gene to be represented. By using an array many transcripts or genes can be determined, preferably, in a single experiment. With the aid of a suitable analyzer, such as an automatic reader device, the amount of target bound to the probes in the array can be precisely measured.

Determining the amount of peptides or polypeptides referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal - may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass-spectrometers, NMR-analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Determination of the amount of a polypeptide, preferably, comprises the use of antibodies that specifically bind to the polypeptide to be determined. Preferably, if the polypeptide to be determined is derived from the translation of a specifically spliced HPV transcript, than the antibody specifically shall bind to the region of the polypeptide that is encoded by the nucleic acids flanking the splice junction. Preferred regions with respect to the gene products mentioned herein to which the antibodies shall specifically bind are those disclosed in table 7 ofEP 2 184 368 A1 (which table is herewith incorporated by reference).

Preferably, for the determination of the amount of the E1C polypeptide (encoded by 880^2582 spliced mRNAs), the antibody shall specifically bind to the E1C polypeptide, in particular to a peptide having an amino acid sequence as shown in SEQ ID NO: 23.

Preferably, for the determination of the amount of the E6*I polypeptide (encoded by 226^409 spliced mRNAs), the antibody shall specifically bind to E6*I polypeptide, in particular to a peptide having an amino acid sequence as shown in SEQ ID NO: 24.

Preferably, for the determination of the amount of the E1^E4 polypeptide (encoded by 880^3358 spliced mRNAs), the antibody shall specifically bind to the E1^E4 polypeptide, in particular to a peptide having an amino acid sequence as shown in SEQ ID NO: 25.

Preferably, for the determination of the amount of the E6*II polypeptide (encoded by 226^526 spliced mRNAs), the antibody shall specifically bind to the E6*II polypeptide, in particular to a peptide having an amino acid sequence as shown in SEQ ID NO:26.

The term "amount" as used herein encompasses the absolute amount of a gene product, the relative amount or concentration of the said gene product as well as any value or parameter which correlates thereto or can be derived therefrom. Such values or parameters comprise intensity signal values from all specific physical or chemical properties obtained from the said gene product by direct measurements. Moreover, encompassed are all values or parameters which are obtained by indirect measurements specified elsewhere in this description. E.g. for polypeptides response levels can be determined from biological read out systems in response to the peptides or intensity signals obtained from specifically bound ligands. It is to be understood that values correlating to the aforementioned amounts or parameters can also be obtained by all standard mathematical operations.

The term "determining the presence or absence of a gene product" is understood by the skilled person. As used herein the term, preferably, relates to assessing whether a gene product is absent or present in a sample. Preferably, the presence of a gene product of E1C in a sample from a subject indicates that said subject suffers from a severe form of HR-HPV infection. Preferably, the absence of a gene product of E1C in a sample from a subject in a sample indicates that said subject suffers from a mild form of HPV infection.

Assessing whether a gene product is present or absent in a sample can be done by well known methods. E.g., if the number of molecules of a gene product is below detection limit, it will be concluded that the gene product is absent; if said number of molecules is above the detection limit, it will be concluded that the gene product is present in the sample. It is to be understood that the detection limit may depend on the type of detection system used; e.g. in PCR-based assays one molecule of a transcript may be detected, whereas in an ELISA assay several polypeptide molecules may be necessary to provide a detectable signal. The person skilled in the art knows how to adjust the detection system employed for maximum sensitivity and reliability, including inclusion of appropriate controls. The method used for determination of the amount of a gene product depends on the nature of the gene product, i.e. whether the gene product is a transcript or a polypeptide.

Determining the presence of the absence of a gene product in a sample can also be done by determining the amount of the gene product in said sample and comparing the, thus determined amount to a reference amount. Determination of the amount of a transcript, and thus the amount of an mRNA, in a sample of a subject can be done by any method deemed appropriate. Preferably, the amount of a transcript is determined by using a probe oligonucleotide that specifically detects the transcript to be analyzed. All methods for determining the amount of a transcript could also be used to determine the presence or absence of a gene product, as described herein above.

The term "comparing" as used herein encompasses comparing the value determined by calculating a ratio of the amount of the gene products herein to a suitable reference source specified elsewhere in this description. It is to be understood that comparing as used herein refers to a comparison of values. The comparison referred to herein may be carried out manually or computer-assisted. For a computer-assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the ratio calculated in the methods of the present invention to a reference ratio it is possible to predict the risk of mortality in a subject suffering from HPV positive OPSCC. Therefore, the reference is to be chosen so that either a difference or a similarity in the compared values allows for predict the risk of mortality in a subject suffering from HPV positive OPSCC.

Accordingly, the term "reference ratio" as used herein, preferably, refers to a value which allows prediction of the risk of mortality in a subject. Accordingly, the ratio may be derived from carrying out steps of the methods of the present invention and calculating a ratio of the amount of the gene products as set forth herein, wherein the sample is derived from a subject known to be at risk of mortality or from a sample from a subject known to be not at risk of mortality. Thus, the reference ratio, preferably, can be derived from a subject known to be at risk of mortality or from a subject known to be not at risk of mortality. Moreover, the subject from whom the reference ratio of the amount of the E6* gene product to the amount of the E1^E4 gene product is derived, preferably, shall comprise the HPV genome in an integrated form, if the ratio of the amount of the E6* gene product to the amount of the E1^E4 calculated. Thus, the HPV genome (or at least parts of the HPV genome, in particular E6 and E7) shall be stably integrated into chromosomal DNA of the subject. In summary, the aforementioned reference ratio, preferably, can be derived from a subject known to be at risk of mortality or from a subject known to be not at risk of mortality, wherein said subject comprises the HPV in an integrated form.

A particularly preferred reference ratio of the amount of the E6* product to the amount of the E1^E4 gene product is 1.5.

Moreover, the references, preferably, define thresholds. Suitable reference ratios or thresholds may be determined by the method of the present invention from a reference sample to be analyzed together, i.e. simultaneously or subsequently, with the test sample. It is to be understood that the value of the reference or threshold may vary depending on the nature of the gene product (transcript or polypeptide) and depending on how the amount of a gene product is determined in the sample. For example, if the determination of the amount of the gene products includes amplification of the gene product by PCR (polymerase chain reaction), the determined amount of a gene product may depend, e.g., on the oligonucleotides used for the PCR reaction since the amplification efficiency of various oligonucleotide pairs for the amplification of a specific gene product varies. However, the person skilled in the art considers this when calculating the reference ratio. Particularly, the person skilled knows that, preferably, the same means and methods have to be used for determining the amounts of a specific gene product in a reference sample and in a test sample.

Preferably, the ratio calculated in step a) of the present invention is the ratio of the amount of the E6* product to the amount of the E1^E4 gene product. It is to be understood, that also the ratio of the amount of the E1^E4 gene product to the E6* product can be calculated. If the ratio of the amount of the E6* product to the amount of the E1^E4 gene product is calculated, the following, preferably, applies as a diagnostic algorithm:
Preferably, the subject is at risk of mortality if
   i) the ratio of the amount of the E6* product to the amount of the E1^E4 gene product is lower than the reference ratio, and
   ii) if in step b) the absence of the E1C gene product is detected.
Preferably, the subject is at not at risk of mortality if
   i) the ratio calculated in step a) is larger than the reference ratio, or
   ii) in step b) the presence of the E1C gene product is detected, or
   iii) the ratio calculated in step a) is larger than the reference ratio and in step b) the presence of the E1C gene product is detected.

In a preferred embodiment of the method of the aforementioned method, step b) is carried out as follows:
b1) determining the amount of an E1C gene product and the amount of a L1 gene product ofHPV in a sample from said subject,
b2) calculating a ratio of the amount of the E1C gene product to the amount of the L1 gene product, and
b3) comparing the, thus, calculated ratio to a reference ratio of the amount of the E1C gene product to the amount of the L1 gene product.

Accordingly, the present invention also envisages a method for predicting the risk of mortality in a subject suffering from oropharyngeal squamous cell cancer (OPSCC), in particular from HPV positive OPSCC, comprising the steps of:
a) determining the amount of an E6* gene product and the amount an E1^E4 gene product of HPV in a sample from said subject, calculating a ratio of the amount of the E6* gene product to the amount of the E1^E4 gene product, and comparing the, thus, calculated ratio to a reference ratio of the amount of the E6* gene product to the amount of the E1^E4 gene product, and
b) determining the amount of an E1C gene product and the amount of a L1 gene product of HPV in a sample from said subject, calculating a ratio of the amount of the E1C gene product to the amount of the L1 gene product, and comparing the, thus, calculated ratio to a reference ratio of the amount of the E1C gene product to the amount of the L1 gene product, whereby the risk of mortality of said subject is to be predicted.

The term "L1 gene product" as used herein, preferably, refers to 3632^5639 spliced transcript of HPV16 or the HPV L1 polypeptide being truncated by 26 amino acids and encoded by said 3632^5639 spliced transcript. The L1 polypeptide of HPV is a capsid protein. During late stages of the productive infection the major capsid protein, the L1 polypeptide is expressed in differentiated cells near the top of the epithelium and forms with L2 polypeptide of HPV 16 the viral capsids in the granular layer. The amino acid sequence of the L1 polypeptide, truncated by 26 amino acids, encoded by said 3632^5639 spliced mRNAs is shown in SEQ ID NO: 27. The sequence of the polynucleotide encoding said L1 polypeptide is shown in SEQ ID NO: 28.

It is particularly envisaged that the L1 gene product is a 3632^5639 spliced transcript, i.e. gene product comprising the 3632^5639 junction. The 3632^5639 spliced transcript, preferably, comprise the nucleic acid sequence that is generated by linking the 3632 donor nucleotide to the 5639 acceptor nucleotide. Accordingly, 3632^5639 spliced mRNAs, preferably, comprise the said nucleic acid sequence with the 3632^5639 splice junction. Preferably, said spliced mRNAs comprise a nucleic acid sequence as shown in SEQ ID NO: 29 (indicated is the corresponding DNA sequence).

The term "reference ratio" has been described elsewhere herein. As set forth above, the term, preferably, refers to a value which allows prediction of the risk of mortality in a subject. Accordingly, the ratio may be derived from carrying out steps of the methods of the present invention and calculating a ratio of the amount of the gene products as set forth herein, wherein the sample is derived from a subject known to be at risk of mortality or from a sample from a subject known to be not at risk of mortality. Thus, the reference ratio, preferably, can be derived from a subject known to be at risk of mortality or from a subject known to be not at risk of mortality. Moreover, the subject from whom the reference ratio is derived, preferably, shall comprise the HPV genome in an episomal form, if the calculated ratio is the ratio of the amount of the E1C gene product to the amount of the L1 gene product, i.e. the HPV genome shall not be integrated into the chromosomal DNA of the subject. In summary, the aforementioned reference ratio, preferably, can be derived from a subject known to be at risk of mortality or from a subject known to be not at risk of mortality, wherein said subject comprises the HPV in an episomal form.

A particularly preferred reference ratio of the amount of the E1C product to the amount of the L1 gene product is 0.003.Preferably, the ratio calculated in step a) of the present invention is the ratio of the amount of the E6* product to the amount of the E1^E4 gene product. Preferably, the ratio calculated in step b) of the present invention is the ratio of the amount of the E1C product to the amount of the L1 gene product.

If the aforementioned ratios are calculated, the following, preferably, applies as a diagnostic algorithm:
Preferably, the subject is at risk of mortality if both the ratios calculated in step a) and b) are lower than the reference ratio.
Preferably, the subject at not at risk of mortality if
   i) the ratio calculated in step a) is larger than the reference ratio, or
   ii) the ratio calculated in step b) is larger than the reference ratio, or
   iii) both the ratios calculated in step a) and b) are larger than the reference ratio.

The definitions given herein above apply mutatis mutandis to the method described.

Moreover, the present invention relates to method for predicting the risk of mortality in a subject suffering from HPV positive oropharyngeal squamous cell cancer, comprising the steps of:
a) determining the copy number of HPV per cancer cell in a sample from said subject, and
b) comparing the copy number of HPV determined in step (a) to a reference copy number, wherein the reference copy number is within a range of 0.5 to 2 copies per cancer cell, whereby the risk of mortality is to be predicted.

Preferably, it is predicting whether the subject is at risk of mortality, or not, by carrying out the further step of c) predicting whether said subject is at risk of mortality, or not, based on the result of the comparison carried out in step b).

The term "predicting the risk of mortality" has been defined elsewhere herein. The definition also applies to the aforementioned method.

The terms "copy number of HPV", or "copies of the HPV genome", preferably, relates to the average copy number of HPV16 genome per OPSCC cancer cell, in particular, per cancer cell comprised by the sample to be tested (see also the explanation of the term "copy number of the HPV genome" given elsewhere herein, the definition applies accordingly. Preferably, the average copy number is the arithmetical average of HPV genomes per cancer cell (comprised by the sample).

The reference copy number shall be within a range of 0.5 to 2 copies per cancer cell. Preferably, the reference copy number is 2.0 copies per cancer cell. More preferably, the reference copy number is 1.5 copies per cancer cell. Most preferably, the reference copy number is 1 copy per cancer cell. It is also contemplated that the reference copy number is 0.5 copies per cancer cell.

The following applies as diagnostic algorithm: Preferably, a decreased copy number in a sample from said subject as compared to the reference copy number indicates that the subject is at risk of mortality, whereas an increased copy number in a sample from said subject as compared to the reference copy number indicates that the subject is not at risk of mortality.

Moreover, the present invention relates to the use of a composition of probe oligonucleotides comprising i) a probe oligonucleotide for the determination of 880^2582 spliced mRNAs, ii) a probe oligonucleotide for the determination of 880^3358 spliced mRNAs, and iii) a probe oligonucleotide for the determination of 226^409 and/or 226^526 spliced mRNAs for predicting the risk of mortality in a subject suffering from HPV (human papillomavirus) positive oropharyngeal squamous cell cancer, in particular from low viral load HPV (human papillomavirus) positive oropharyngeal squamous cell cancer.

Furthermore, the present invention relates to the use of a composition of oligonucleotides comprising i) oligonucleotides for the amplification of 880^3358 spliced mRNAs, ii) oligonucleotides for the amplification of 226^409 spliced mRNAs and/or oligonucleotides for the amplification of 226^526 spliced mRNAs, and iii) oligonucleotides for the amplification of 880^2582 spliced mRNAs for predicting the risk of mortality in a subject suffering from HPV (human papillomavirus) positive oropharyngeal squamous cell cancer, in particular from low viral load HPV (human papillomavirus) positive oropharyngeal squamous cell cancer.

Also the present invention relates to the use (i.e. the combined use) of the aforementioned composition of oligonucleotides and the aforementioned composition of probe oligonucleotides for predicting the risk of mortality in a subject suffering from HPV (human papillomavirus) positive oropharyngeal squamous cell cancer, in particular from low viral load HPV (human papillomavirus) positive oropharyngeal squamous cell cancer.

Moreover, the present invention relates to the use of a composition of probe oligionucleotides comprising i) a probe oligonucleotide for the determination of 880^2582 spliced mRNAs, ii) a probe oligonucleotide for the determination of 880^3358 spliced mRNAs, iii) a probe oligonucleotide for the determination of 226^409 and/or 226^526 spliced mRNAs, and iv) a probe oligonucleotide for the determination of 3632^5639 spliced mRNAs for predicting the risk of mortality in a subject suffering from HPV (human papillomavirus) positive oropharyngeal squamous cell cancer, in particular from low viral load HPV (human papillomavirus) positive oropharyngeal squamous cell cancer.

Furthermore, the present invention relates to the use of a composition of oligonucleotides comprising i) oligonucleotides for the amplification of 880^3358 spliced mRNAs, ii) oligonucleotides for the amplification of 226^409 spliced mRNAs and/or oligonucleotides for the amplification of 226^526 spliced mRNAs, iii) oligonucleotides for the amplification of 880^2582 spliced mRNAs, and iv) oligonucleotides for the amplification of 3632^5639 spliced mRNAs, for predicting the risk of mortality in a subject suffering from HPV (human papillomavirus) positive oropharyngeal squamous cell cancer, in particular from low viral load HPV (human papillomavirus) positive oropharyngeal squamous cell cancer.

Also the present invention relates to the use (i.e. the combined use) of the aforementioned composition of oligonucleotides and the aforementioned composition of probe oligonucleotides for predicting the risk of mortality in a subject suffering from HPV (human papillomavirus) positive oropharyngeal squamous cell cancer, in particular from low viral load HPV (human papillomavirus) positive oropharyngeal squamous cell cancer.

All references cited in this specification are herewith incorporated by reference with respect to their entire disclosure content and the disclosure content specifically mentioned in this specification.

The Figures show
**Figure 1****. Overall flow-chart showing the grouping of OPSCC in relation to DNA, viral load and RNA status.** HPV⁻, OPSCC negative for HPV DNA; HPV⁺, OPSCC positive for HPV16 DNA determined by BSGP5+6+-PCR/MPG analysis; HPV_{low}/HPV_{high}, OPSCC with low/high viral load determined by qPCR; RNA⁻, OPSCC positive for HPV16 DNA, but negative for HPV16 E6*II/E6*I RNA; RNA⁺/CxCa⁻, OPSCC positive for HPV16 DNA and for HPV16 E6*II/E6*I RNA, but without CxCa-like viral RNA patterns; RNA⁺/CxCa⁺, OPSCC positive for HPV16 DNA and for HPV16 E6*II/E6*I RNA with CxCa-like viral RNA patterns.

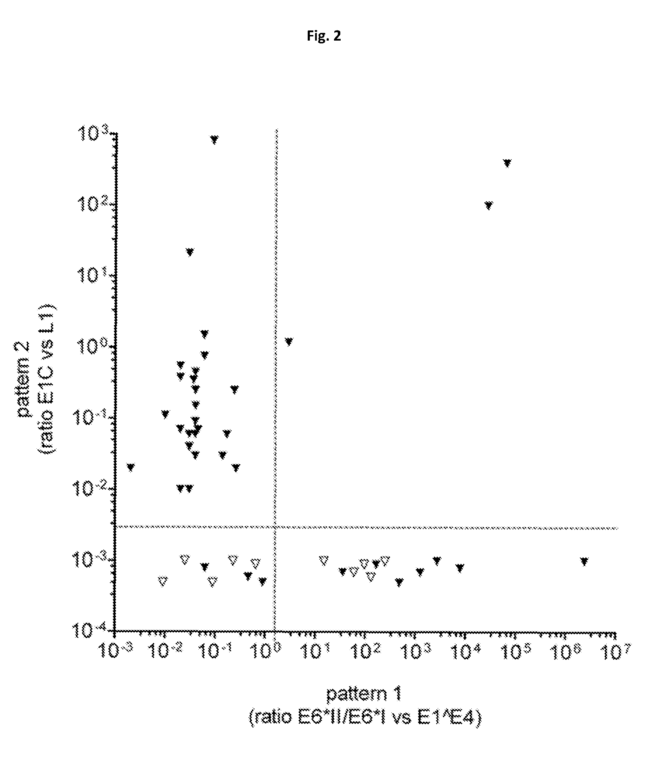

Figure 2. Viral RNA patterns in HPV16 E6*II/E6*I positive OPSCC analogous to CxCa (n=48). Tumors with pattern 1 are located in the lower right quadrant (n=12), tumors with pattern 2 in the upper left (n=25), tumors with both patterns 1 and 2 in the upper right (n=3), and tumors without CxCa-like viral RNA patterns in the lower left quadrant (n=8). Closed triangles, OPSCC with high viral load; open triangles, OPSCC with low viral load. Dashed lines represent thresholds for transcript ratios.

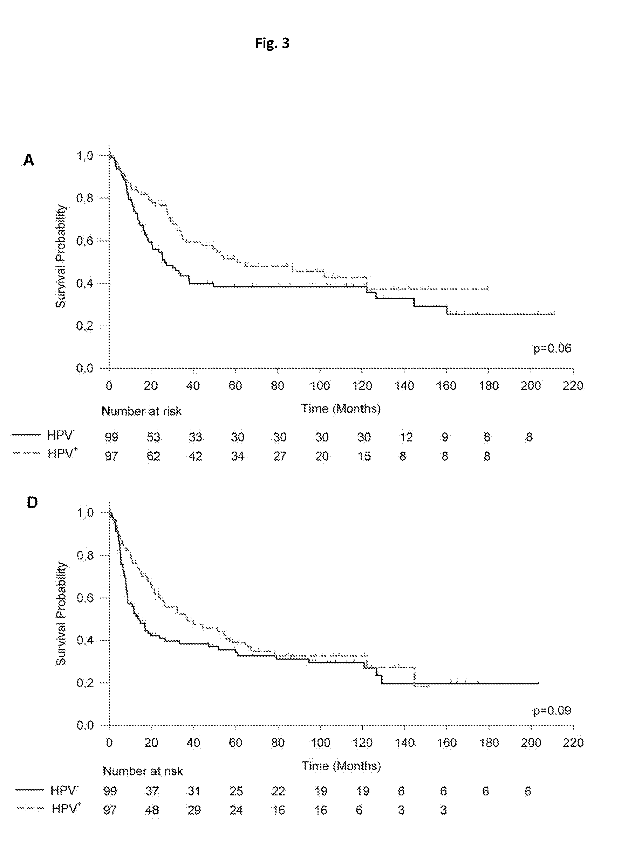

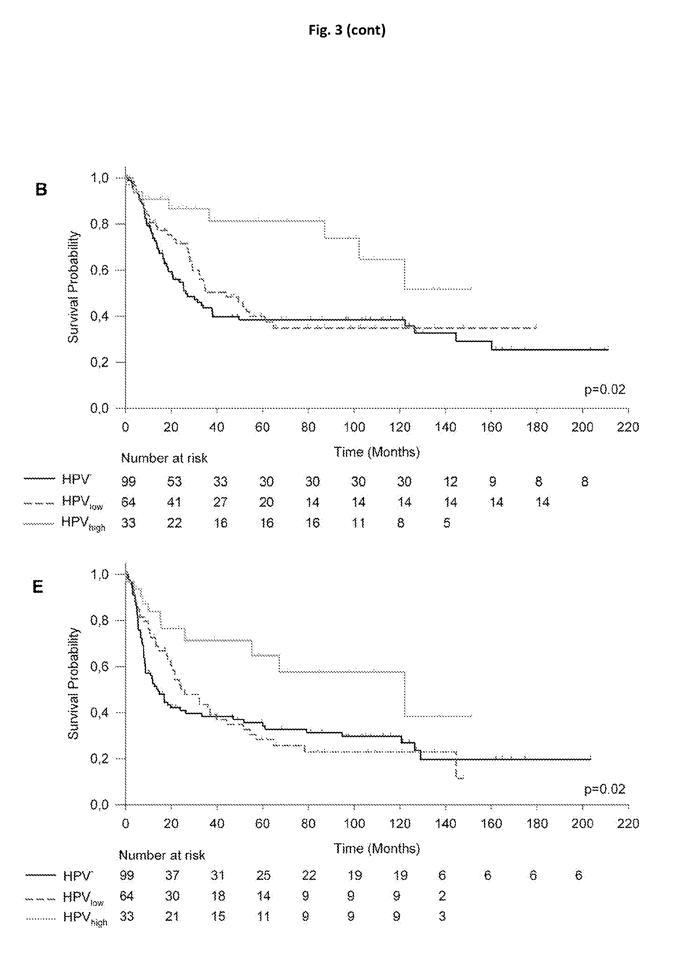

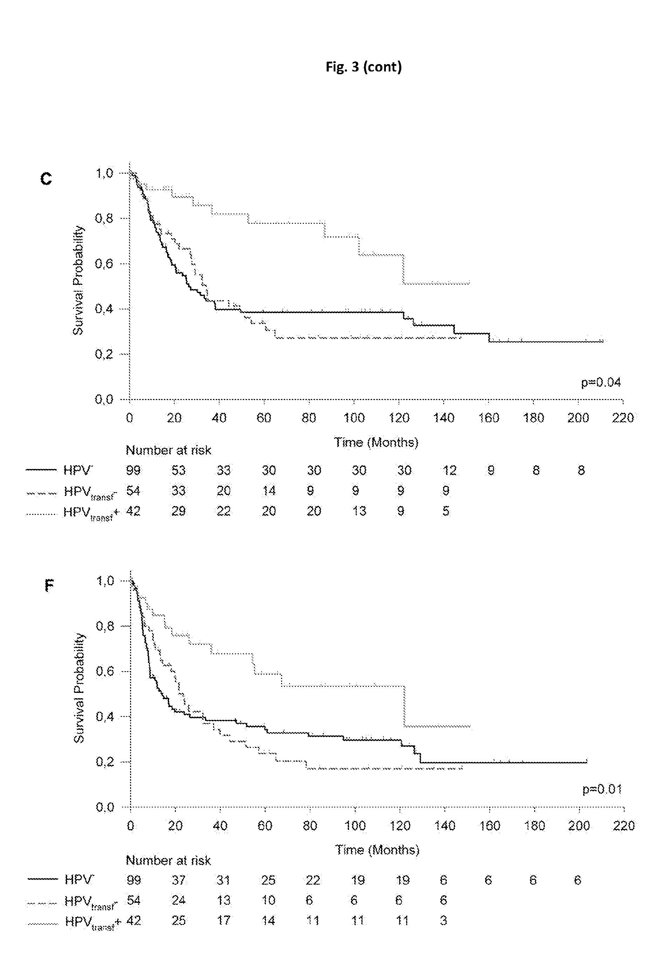

Figure 3. Overall (A, B, C) and progression-free survival (D, E, F) in relation to HPV16 status. The differences between survival curves were assessed by Log-rank test. Tumors were grouped by HPV16 DNA status (A, D), viral load (B, E) and marker combinations (HPV_{transf}⁺, HPV_{high} tumors combined with HPV_{low} tumors positive for CxCa-like viral RNA patterns; HPV_{transf}⁻, HPV_{low} tumors without CxCa-like viral RNA-patterns; C, F). Note that survival was not significantly different if only HPV16 DNA status was assessed. Grouping by viral load status or by marker combinations revealed significant differences in OS and PFS. HPV⁻/HPV⁺, OPSCC negative/positive for HPV16 DNA; HPV_{low}/HPV_{high}, OPSCC with low/high viral load.

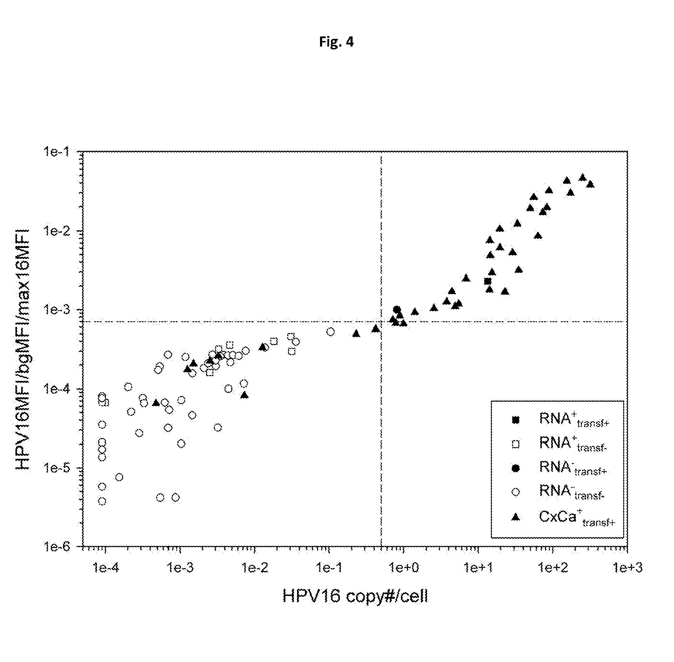

**Figure 4****. Correlation between HPV16 DNA copy numbers determined by qPCR and estimated HPV16 viral load using BSGP5+/6+-PCR/MPG in OPSCC (n=97).** Each symbol depicts one tumor; HPV_{transf}⁻ and HPV_{transf}⁺ tumors are displayed as open and closed symbols, respectively. Tumors were either positive for CxCa-like viral RNA patterns (RNA⁺/CxCa⁺, closed triangles) or positive for viral E6*II/E6*I only (RNA⁺, squares). Circles denote E6*II/E6*I-negative tumors (RNA⁻). Dashed lines represent cutoffs between low viral load (HPV_{low}) and high viral load (HPV_{high}) groups for each analysis (CO_{BSGP5+/6+-PCR/MPG} 7x10⁻⁴; CO_{copy#/cell} 0.5). Note that 9/53 HPV_{low} tumors had CxCa-like viral RNA patterns; one HPV_{high} tumor was RNA-negative. Viral load values (y-axis) analyzed by BSGP5+/6+-PCR/MPG correlated significantly with viral DNA copy numbers (x-axis) resulting from qPCR analysis (r=0.93).

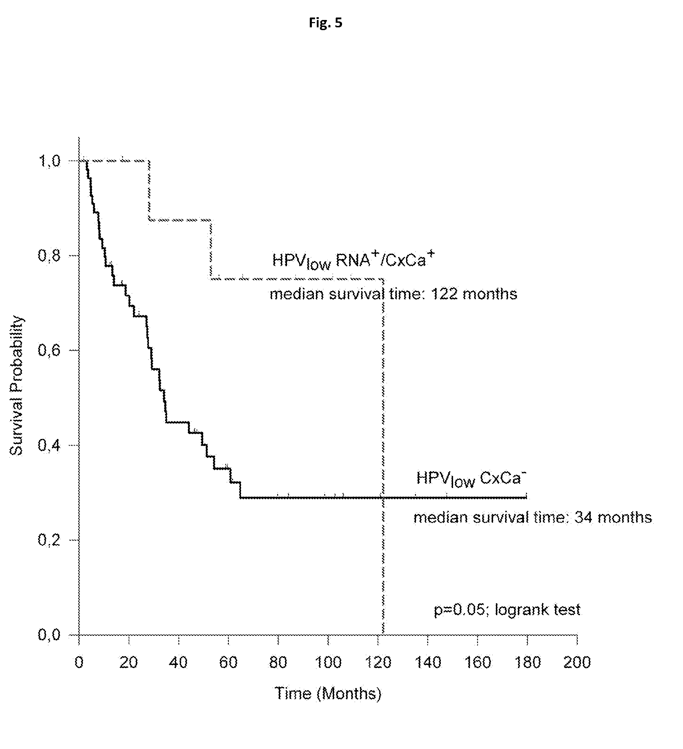

**Figure 5****. Overall survival for patients with low viral load OPSCC in relation to CxCa-like viral RNA patterns.** The differences between survival curves were assessed by Log-rank test. Tumors were grouped by HPV16 CxCa-like viral RNA patterns (HPV_{low}RNA⁺/CxCa⁺, OPSCC with low viral load and with CxCa-like viral RNA patterns; HPV_{low}CxCa⁻, OPSCC with low viral load, but without CxCa-like viral RNA patterns). HPV_{low}RNA⁺/CxCa⁺ patients had a significant better overall survival and a longer median survival time compared to HPV_{low}CxCa⁻ patients.

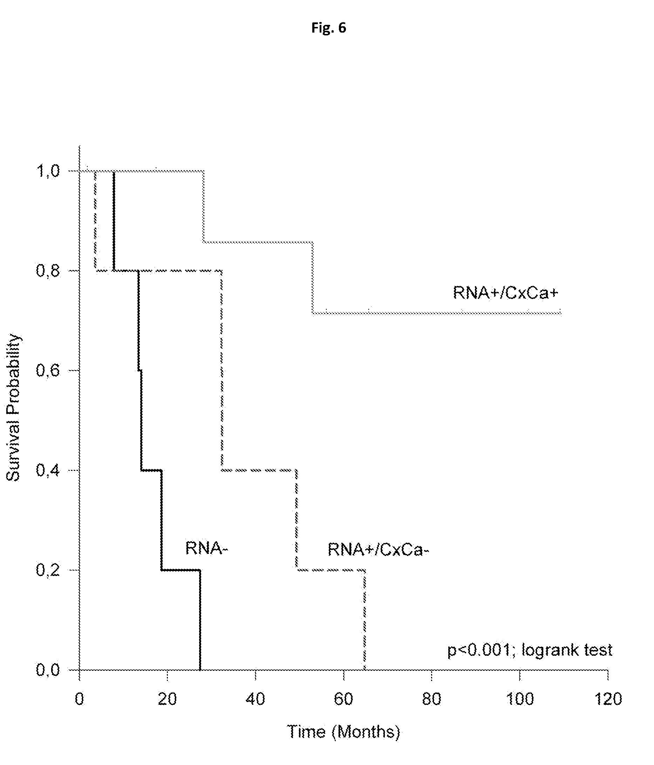

**Figure 6****. Overall survival for case study.** Only patient with low viral load (HPV_{low}) OPSCC were included: 5 patients negative for E6*II/E6*I RNA (RNA⁻), 5 positive for E6*II/E*I, but without CxCa-like viral RNA-patterns (RNA⁺/CxCa⁻) and all 9 with CxCa-like viral RNA patterns (RNA⁺/CxCa⁺). The differences between survival curves were assessed by Log-rank test. Tumors were grouped by HPV16 CxCa-like viral RNA patterns (RNA⁺/CxCa⁺, OPSCC with CxCa-like viral RNA patterns; RNA⁺/CxCa⁻, OPSCC without CxCa-like viral RNA patterns; RNA⁻, OPSCC negative for E6*II/E6*I RNA). RNA⁺/CxCa⁺ patients had a significant better overall survival compared to RNA⁺/CxCa⁻ or RNA⁻ patients, respectively.

The following Examples shall merely illustrate the invention. They shall not be construed, whatsoever, to limit the scope of the invention.

### Example 1: Patients and tissue samples

199 OPSCC patients treated at the department of otolaryngology-head and neck surgery of the University Hospital Heidelberg, Germany, between 1990 and 2008 were included. The study was approved by the Ethics Committee of the Medical Faculty of the University of Heidelberg, study code 193/2003. Biopsies were directly snap-frozen in pre-cooled isopentane/liquid nitrogen and stored at -80°C until further processing. For nucleic acid extractions, cryo-sections of 16 µm thickness yielding between 2 and 10 mg of tissue were cut, homogenized in liquid nitrogen and stored at -80°C. To verify the presence and to estimate the content of tumor cells in cryosections, adjacent sections were stained with haematoxylin & eosin. The mean tumor cell content was 55% (range 25-90%). After each biopsy, the cryostat was cleaned with acetone and ethanol and new gloves, forceps and blades were used. For each homogenization a fresh pistil was used. As negative controls two mucosal biopsies from healthy patients were processed in the same way. Patient and tumor characteristics were obtained from clinical records. Overall survival (OS) was measured as the time from the date of primary tumor diagnosis to the date of OPSCC related death. Survival times of patients who were alive at the date of last follow-up or were dead due to causes other than OPSCC were censored. Progression-free survival (PFS) time was calculated from the date of primary tumor diagnosis to the date of the first local recurrence, lymph node or distant metastasis, second primary carcinoma or date of OPSCC related death (events), or to the date of last follow-up without progression (censored).

### Example 2: Viral load analysis

DNA was isolated using Qiagen's QIAamp DNA Mini Kit. The BSGP5+/6+-PCR/MPG assay comprises the BSGP5+/6+-PCR generating biotinylated amplimers of ~150 bp from the HPV L1 region followed by a genotyping assay with bead-based Luminex suspension array technology, which is able to simultaneously identify 27 mucosal HPV types (Schmitt et al., 2006; Schmitt et al., 2008). The assay co-amplifies cellular beta-globin sequences as internal DNA quality control. Median fluorescence intensities (MFI) of HPV16 plasmid standards with one HPV16 genome copy per 100 ng human placental DNA were taken as cutoff for HPV16 DNA positivity. Quantification of HPV 16 signals was accomplished by calculating for each positive reaction the relative HPV16 MFI signal (%) by dividing the measured HPV16 MFI value with the maximum HPV16 value. Finally, the relative MFI (%) was divided by the measured β-globin MFI value to form a non-descriptive viral load value (%HPVMFI / β-globin MFI). High viral load was assessed by a predefined high viral load cutoff (0.0007 units) for Hr-HPV types (Schmitt et al., in preparation). This high viral load cutoff has been optimized to distinguish smears with normal cytology from those with cervical abnormalities. Tumors positive for HPV16 DNA by BSGPS+/6+-PCR/MPG (HPV+) were further analyzed using quantitative real-time PCR (qPCR) targeting HPV16-specific sequences of the E6 gene to obtain viral load values (number of HPV copies/cell) and an objective high viral load cutoff. Because on average the specimens contained approx. 50% tumor cells, we defined 0.5 copies/cell as cutoff for a high viral load. The real-time quantitative PCR (qPCR) was performed under similar conditions described elsewhere (Depuydt CE, Benoy IH, Bailleul EJ, Vandepitte J, Vereecken AJ, Bogers JJ. Improved endocervical sampling and HPV viral load detection by Cervex-Brush Combi. Cytopathology 2006;17(6):374-81). qPCR for β-globin was used to verify the quality of DNA in the sample and to measure the amount of input DNA. Viral loads in each specimen were expressed as the number of HPV copies/cell.

All 199 OPSCC biopsies yielded DNA of good quality with the beta-globin gene co-amplified in each sample. Of 100 OPSCC (50%) positive for HPV DNA analyzed by BSGP5+/6+-PCR/MPG, 97 contained HPV16 DNA (HPV⁺). Three tumors contained a single HPV-genotype, HPV18, 33 or 35; these were not further analyzed here. No multiple infections were detected. HPV16 DNA prevalence increased from 37% in tumors collected between 1990 and 1999 to 63% in the tumors collected from 1999 to 2008. To confirm the quantitative BSGP5+/6+-PCR/MPG results, all HPV⁺ tumors were again analyzed by qPCR. 11 tumors with low viral load by BSGP5+/6+-PCR/MPG were negative by qPCR, 86 were HPV16 DNA-positive resulting in 89% concordance (r=0.93, p<.01; Spearman's rank correlation). Of the 97 HPV⁺ tumors, 64 (66%) had a low (HPV_{low}) and 33 (34%) had a high viral load (HPV_{high}). Cox proportional hazard models adjusted for gender, age, clinical stage, alcohol/tobacco consumption and first-line therapy revealed a statistically significant association of HPV viral load with both OS and PFS in comparison to the HPV⁻ group. While DNA-positivity was not related with a statistically significant change in hazards, HPV_{high} patients had a 67% lower risk of death from OPSCC. The hazard ratio with respect to PFS was HR=0.46 for HPV_{high} patients compared to patients with HPV⁻ OPSCC(Table 1).

**Table 1. Multivariate analysis of overall and progression-free survival**

| | | | | Overall survival | | | Progression-free survival | | |
|---|---|---|---|---|---|---|---|---|---|
| | Parameter | | | HR | 95% CL | p-value | HR | 95% CL | p-value |
| Demographic, clinical and behavioral factors | | | | | | | | | |
| | Age | | | 1.01 | 0.81-1.25 | 0.9 | 1.01 | 0.83-1.23 | 0.9 |
| | Gender (female vs male) | | | 1.09 | 0.66-1.77 | 0.7 | 0.93 | 0.60-1.44 | 0.7 |
| | Clinical stage (IV vs I-III) | | | **1.79** | **1.03-3.12** | **0.04** | 1.30 | 0.82-2.07 | 0.3 |
| | Therapy status (R/C vs surgery) | | | **2.25** | **1.42-3.57** | **<0.001** | **1.89** | **1.22-2.91** | **0.004** |
| | Tobacco | *current vs never* | | 2.09 | 0.83-5.30 | | **2.52** | **1.08-5.92** | |
| | | *former vs never* | | 1.27 | 0.40-4.01 | 0.1 | 1.39 | 0.49-3.90 | 0.03 |
| | Alcohol | | | | | | | | |
| | | *current vs never* | | 1.22 | 0.48-3.10 | | 1.10 | 0.49-2.56 | |
| | | *former vs never* | | 1.24 | 0.42-3.62 | 0 9 | 1.18 | 0.45-3.11 | 0.9 |
| Single HPV markers | | | n | | | | | | |
| | HPV⁻ (reference) | | 99 | 1.00 | | | 1.00 | | |
| | HPV⁺ | | 97 | 0.68 | 0.45-1.04 | 0.07 | 0.77 | 0.53-1.12 | 0.2 |
| | HPV⁻ (reference) | | 99 | 1.00 | | | 1.00 | | |
| | HPV_{low} | | 64 | 0.82 | 0.53-1.26 | | 0.88 | 0.60-1.30 | |
| | HPV_{high} | | 33 | **0.33** | **0.15-0.76** | **0.03** | **0.46** | **0.23-0.92** | 0.09 |
| | HPV⁻ (reference) | | 99 | 1.00 | | | 1.0 | | |
| | RNA⁻ | 48 | 0.90 | 0.56-1.44 | | 0.90 | 0.58-1.40 | | |
| | RNA⁺ | 48 | 0.48 | **0.26-0.89** | 0.06 | 0.63 | 0,37-1.06 | 0.2 | |
| | HPV⁻ (reference) | 99 | 1.00 | | | 1.00 | | | |
| | RNA⁻ plus RNA⁺/CxCa⁻ | 56 | 0.95 | 0.61-1.48 | | 0.93 | 0.61-1.40 | | |
| | RNA⁺/CxCa⁺ | 40 | **0.31** | **0.14-0.68** | **0.01** | **0.51** | **0.27-0.95** | 0.1 | |
| HPV marker combinations | | | | | | | | | |
| | HPV⁻ (reference) | 99 | 1.00 | | | 1.00 | | | |
| | HPV_{transf}⁻ | 54 | 0.96 | 0.61-1.50 | | 0.95 | 0.63-1.42 | | |
| | HPV_{transf}⁺ | 42 | **0.33** | **0.15-0.69** | **0.01** | **0.50** | **0.27-0.92** | 0.08 | |
| HR, hazard ratio; CL, confidence limits. HR for age, gender, clinical stage, therapy status, alcohol and tobacco consumption were calculated using a Cox model including only these factors. HR for single HPV markers were computed using four different models: i) DNA model (HPV⁺ (n=97), HPV16 DNA-positive OPSCC), ii) viral load model (HPV_{low} (n=64), OPSCC with low viral load; HPV_{high} (n=33), OPSCC with high viral load), iii) RNA models (RNA⁻ (n=48), DNA-positive/RNA-negative OPSCC; RNA⁺ (n=48), DNA-positive/RNA-positive OPSCC or iv) RNA pattern model (RNA⁻ plus RNA⁺/CxCa⁻ (n=56), DNA-positive/RNA-negative OPSCC plus DNA-positive/RNA-positive OPSCC without CxCa-like viral RNA patterns; RNA⁺/CxCa⁺ (n=40), DNA-positive/RNA-positive with CxCa-like viral RNA patterns). HR were also calculated for HPV marker combinations: HPV_{transf}⁻ (n=54), OPSCC with low viral load and without CxCa-like viral RNA patterns; HPV_{transf}⁺ (n=42), OPSCC with high viral load and/or CxCa-like viral RNA patterns. HPV⁻ group (HPV16 DNA-negative OPSCC) with n=99 resulting from BSGP5+/6+-PCR/MPG analysis was reference category for all models. Models were adjusted by age, gender, clinical stage, therapy status and alcohol/tobacco consumption. Statistically significant values are in bold. | | | | | | | | | |

### Example 3: RNA pattern analysis

HPV16 DNA-positive OPSCC determined by BSGP5+/6+-PCR/MPG were analyzed for viral RNA patterns. RNA was isolated using Qiagen's RNeasy Mini Kit. DNase I digestion (Qiagen) was included to ensure an exclusive amplification of RNA. Nucleic acid sequence-based amplification (NASBA) and hybridization to splice-specific probes on Luminex beads were carried out as described (Schmitt et al., 2010 and EP 2 184 368 A1). For quantification of E6*II RNA, a calibrator RNA was competitively co-amplified in the same reaction and used to normalize the E6*II MFI values. E6*I copy numbers in E6*II-negative samples were estimated by the same calibrator RNA. E6*I/E6*II positive tumors (RNA⁺) were further examined quantitatively for viral transcripts E1C (880^2582), E1^E4 (880^3358) and L1 (3632^5639) using their respective calibrators. For absolute quantification, each experiment included external standard dilution series of *in vitro* RNA. Transcript ratios were calculated for E6*II or E6*I over E1^E4 and for E1C over L1. Tumors with either an E6*/E1^E4 ratio of > 1.5 (pattern 1) and/or an E1C/L1 ratio of >0.003 (pattern 2) were classified as CxCa-like (RNA⁺/CxCa⁺). RNA of one healthy mucosa and of one HPV⁻ tumor served as negative controls. All RNA⁻ samples were positive for the ubiquitin C transcript and the controls were negative for all viral transcripts.

Of 48 RNA⁺ tumors 40 (83%) displayed CxCa-like viral RNA patterns (RNA⁺/CxCa⁺, Figure 1). Of these, 12 (25%) displayed pattern 1 (E6/E1^E4 ratio > 1.5), 25 (52%) pattern 2 (E1C/L1 ratio > 0.003), and 3 (6%) displayed both patterns (Figure 2). The 8 tumors not displaying CxCa-like viral RNA patterns (RNA⁺/CxCa⁻) exhibited very low signals for all viral transcripts. Of 33 HPV_{high} OPSCC 31 (94%) displayed CxCa-like viral RNA patterns. However, 9/64 (14%) HPV_{low} tumors also displayed CxCa-like viral RNA-patterns (Figure 1). Cox proportional hazards models of HPV markers adjusting for demographic, clinical and behavioral factors revealed a statistically significant association of viral RNA patterns with both OS and PFS. While DNA-positivity was not related with a statistically significant change in hazards, RNA⁺/CxCa⁺ patients had a 69% lower risk of death from OPSCC. The hazard ratio with respect to PFS was HR=0.51 for RNA⁺/CxCa⁺ patients compared to patients with HPV⁻ OPSCC (Table 1).

### Example 4: Viral marker combinations for patients with low viral loads

The groups HPV_{high} and RNA⁺/CxCa⁺ were well correlated as 31/33 (94%) HPV_{high} tumors were RNA⁺/CxCa⁺. However, 9 RNA⁺/CxCa⁺ tumors were not detected by the marker HPV_{high} (Figure 1). Assuming that RNA⁺/CxCa⁺ was a critical parameter for tissues transformed by HPV16 (HPV_{transf}), we combined the HPV_{high} group with the RNA⁺/CxCa⁺ cases among the HPV_{low} group to form the HPV_{transf}⁺ group, whereas HPV_{low} tumors without CxCa-like viral RNA formed the HPV_{transf}⁻ group.

Prevalence of HPV_{transf}⁺ tumors did not change significantly over time, in contrast to the prevalence of HPV⁺. The HPV_{transf}⁺ group (HPV_{high} and/or RNA⁺/CxCa⁺) had a comparable survival advantage as the single marker groups HPV_{high} and RNA⁺/CxCa⁺ (Table 1). Importantly, tumors without transcriptionally active HPV16 represented by the HPV_{transf}⁻ group conferred similar risks for OPSCC cancer death and tumor relapse as patients with HPV⁻ tumors (Table 1). Compared with the Cox model of demographic, clinical and behavioral factors alone prediction accuracy of overall survival was always improved by including HPV marker data into the model. The strongest improvement resulted from using the HPV16 marker combinations defined above. No improvement was seen for predicting PFS by combining clinical data with single HPV markers. But again, prediction accuracy was enhanced using a Cox model including HPV16 marker combinations.

By HPV DNA status only, HPV⁺ and HPV⁻ tumors were marginally distinct entities, since they showed similar survival curves, and in multivariate Cox proportional hazard models, patients with HPV⁺ tumors had no statistically significantly better outcome in OS and PFS compared to patients with HPV⁻ tumors (Table 1). However, expression of the viral E6*I/E6*II transcripts (RNA⁺ group) was superior in defining patients with better survival, a finding in full agreement with a recent French study (Jung et al., 2010). Moreover, a high viral load or the expression of CxCa-like viral RNA patterns were even better markers for improved patient survival resulting in comparable HRs. The marker RNA⁺/CxCa⁺ was most sensitive since it detected 32/33 HPV_{high} tumors and 9 HPV_{low} tumors (n=41) (Table 1). However, the analysis of CxCa-like viral RNA patterns is very complex and to date not well feasible for every clinical laboratory. A feasible and precise algorithm to identify truly HPV-driven OPSCC was provided by taking the HPV_{high} tumors which showed a very good correlation with RNA⁺/CxCa⁺ tumors (Figure 1), and combining them with HPV_{low} tumors positive for CxCa-like RNA patterns. With this algorithm, analyses of RNA patterns can be limited to HPV_{low} tumors, without losing sensitivity. The HR of the HPV_{transf}⁺ group was comparable to the best single marker RNA⁺/CxCa⁺. A third alternative algorithm may consist of the detection of high viral load only. The HR of the HPV_{high} group was comparable to the best single marker RNA⁺/CxCa⁺. This algorithm should be favorable if only low technical setting are available and the absolute sensitivity of the RNA⁺/CxCa⁺ marker is not required.

In conclusion, by using highly sensitive HPV DNA and RNA detection technologies, we not only detected OPSCC with high viral load and with CxCa-like viral RNA patterns, but also a fraction of tumors with low viral load in which the transcriptional activity of the virus indicated a carcinogenic role. These tumors represent a clinically distinct subgroup with significantly better OS and PFS. Patients with HPV-driven OPSCC have been shown to have better survival even with radiochemotherapy as primary treatment modality. In situations like in Germany, with heterogeneity among the HPV DNA-positive tumor patients, precise definition of HPV transcriptional activity in the tumor as suggested here could help in selection of the primary treatment modality.

### Example 5: Case study

Of the low viral load group (HPV_{low}), five patients negative for E6*II/E6*I RNA (RNA⁻), five patients positive for E6*II/E*I but without CxCa-like viral RNA-patterns (RNA⁺/CxCa⁻) and all nine patients with CxCa-like viral RNA patterns (RNA⁺/CxCa⁺) were included in this study. There were only 2/9 (22%) tumor-related deaths and only 4/9 (44%) progression events among the RNA⁺/CxCa⁺ patients, whereas all of the RNA⁻ and RNA⁺/CxCa⁻ patients died due to OPSCC and all patients suffered from tumor progression (Table 2). The median survival time was 14 and 32 month for RNA⁻ and RNA⁺/CxCa⁻ patients, respectively. In contrast, the median survival time for RNA⁺/CxCa⁺ patients was not reached (Figure 6).

**Table 2: Case study of patients with low viral load OPSCC.**

| ID | HPV16 copies/cell | RNA group | Gender | Age (years) | OS status | OS (months) | PFS status | PFS (months) |
|---|---|---|---|---|---|---|---|---|
| 1195 | 0.0003 | RNA⁻ | 1 | 47 | 1 | 7.8 | 1 | 5.2 |
| 294 | 0.001 | RNA⁻ | 1 | 67 | 1 | 13.4 | 1 | 8.1 |
| 89 | 0.001 | RNA⁻ | 2 | 57 | 1 | 14.0 | 1 | 10.5 |
| 1494 | 0.003 | RNA⁻ | 2 | 53 | 1 | 18.6 | 1 | 11.0 |
| 47 | 0.04 | RNA⁻ | 1 | 40 | 1 | 27.4 | 1 | 25.9 |
| 678 | 0.005 | RNA⁺/CxCa⁻ | 1 | 67 | 1 | 32.4 | 1 | 24.1 |
| 1051 | 0.02 | RNA⁺/CxCa⁻ | 1 | 44 | 1 | 49.3 | 1 | 44.2 |
| 1265 | 0.0001 | RNA⁺/CxCa⁻ | 1 | 57 | 1 | 32.2 | 1 | 32.2 |
| 1284 | 0.03 | RNA⁺/CxCa⁻ | 1 | 52 | 1 | 3.6 | 1 | 3.6 |
| 1285 | 0.003 | RNA⁺/CxCa⁻ | 1 | 59 | 1 | 64.7 | 1 | 64.7 |
| 65 | 0.001 | RNA⁺/CxCa⁺ | 2 | 76 | 0 | 86.9 | 0 | 86.9 |
| 91 | 0.002 | RNA⁺/CxCa⁺ | 1 | 49 | 0 | 109.1 | 0 | 109.1 |
| 122 | 0.003 | RNA⁺/CxCa⁺ | 1 | 57 | 0 | 56.1 | 1 | 54.4 |
| 679 | 0.003 | RNA⁺/CxCa⁺ | 1 | 65 | 1 | 28.2 | 1 | 3.1 |
| 1081 | 0.007 | RNA⁺/CxCa⁺ | 1 | 68 | 1 | 52.9 | 1 | 18.4 |
| 1341 | 0.0005 | RNA⁺/CxCa⁺ | 1 | 54 | 0 | 65.7 | 0 | 65.7 |
| 1523 | 0.01 | RNA⁺/CxCa⁺ | 1 | 69 | 0 | 17.4 | 0 | 17.4 |
| 1553 | 0.2 | RNA⁺/CxCa⁺ | 1 | 72 | 0 | 101.9 | 1 | 36.0 |
| 1643 | 0.4 | RNA⁺/CxCa⁺ | 2 | 54 | 0 | 1.8 | 0 | 1.8 |

| | | | | | | | | |
|---|---|---|---|---|---|---|---|---|
| RNA⁻, OPSCC negative for E6*II/E6*I transcripts; RNA⁺/CxCa⁻, OPSCC positive for E6*II/E6*I transcripts, but without CxCa-like viral RNA patterns RNA⁺/CxCa⁺, OPSCC positive for E6*II/E6*I transcripts with CxCa-like viral RNA patterns; Gender: 1, male; 2, female; OS/PFS status: 0, censored; 1, event | | | | | | | | |

## Claims

1. A method for predicting the risk of mortality in a subj ect suffering from low viral load HPV (human papillomavirus) positive oropharyngeal squamous cell cancer, comprising the steps of:
a) determining the amount of an E6* gene product and the amount of an E1^E4 gene product of HPV in a sample from said subject, calculating a ratio of the amount of the E6* gene product to the amount of the E1^E4 gene product, and comparing the, thus, calculated ratio to a reference ratio of the amount of the E6* gene product to the amount of the E1^E4 gene product, and
b) determining the presence or absence of an E1C gene product in a sample from said subject,
whereby the risk of mortality in said subject is to be predicted.

2. The method of claim 1, wherein the subject suffering from low viral load HPV positive oropharyngeal squamous cell cancer, comprises between 0.001 and 1 copy of HPV per cancer cell.

3. The method of claim 1 or 2, wherein the E6* gene product is an E6*I or E6*II gene product.

4. The method of any one of claims 1 to 3, wherein the gene product is a transcript.

5. The method of any one of claims 1 to 4, wherein the E1C gene product are spliced transcripts comprising the 880^2582 junction, wherein the E6* gene product are spliced transcripts comprising the 226^409 junction and/or spliced transcripts comprising the 226^526 junction, and/or wherein the E1^E4 gene product are spliced transcripts comprising the 880^3358 junction.

6. The method of any one of claims 1 to 5, wherein the HPV is HPV16.

7. The method of any one of claims 1 to 6, wherein the test sample is a biopsy of a squamous cell carcinoma of the oropharynx.

8. The method of any one of claims 1 to 7, wherein the subject is at risk of mortality if the ratio calculated in step a) is lower than the reference ratio and if in step b) the absence of the E1C gene product is detected.

9. The method of any one of claims 1 to 8, wherein the subject is not at risk of mortality if
i. the ratio calculated in step a) is larger than the reference ratio,
ii. in step b) the presence of the E1C gene product is detected, or
iii. the ratio calculated in step a) is larger than the reference ratio and in step b) the presence of the E1C gene product is detected.

10. The method of any one of claims 1 to 7, wherein in step b) the amount of an E1C gene product and the amount of a L1 gene product of HPV in a sample from said subject is determined, and wherein a ratio of the amount of the E1C gene product to the amount of the L1 gene product is calculated, and wherein the, thus, calculated ratio is compared to a reference ratio of the amount of the E1C gene product to the amount of the L1 gene product.

11. The method of claim 9, wherein the subject is at risk of mortality if both the ratios calculated in step a) and b) are lower than the reference ratio, or
wherein the subject is at not at risk of mortality if the ratio calculated in step a) is larger than the reference ratio, or if the ratio calculated in step b) is larger than the reference ratio, or if both the ratios calculated in step a) and b) are larger than the reference ratio.

12. A method for predicting the risk of mortality in a subject suffering from HPV (human papillomavirus) positive oropharyngeal squamous cell cancer, comprising the steps of:
a) determining the copy number of HPV per cancer cell in a sample from said subject, and
b) comparing the copy number of HPV determined in step (a) to a reference copy number, wherein the reference copy number is within a range of 0.5 to 2.0 per cancer cell,
whereby the risk of mortality is to be predicted.

13. The method of claim 12, wherein the reference copy number is 1 copy per cancer cell.

14. The method of claim 12 or 13, wherein a decreased copy number in a sample from said subject as compared to the reference copy number indicates that the subject is at risk of mortality, wherein an increased copy number in the sample from said subject as compared to the reference copy number indicates that the subject is not at risk of mortality.

15. Use of a composition of oligonucleotides comprising i) oligonucleotides for the amplification of 880^3358 spliced mRNAs, ii) oligonucleotides for the amplification of 226^409 spliced mRNAs and/or oligonucleotides for the amplification of 226^526 spliced mRNAs, and iii) oligonucleotides for the amplification of 880^2582 spliced mRNAs for predicting the risk of mortality in a subject suffering from HPV (human papillomavirus) positive oropharyngeal squamous cell cancer.


SEQUENCE LISTING

<110>  DKFZ

<120>  Means and methods for predicting the risk of mortality of
       patients with HPV positive oropharyngeal squamous cell cancer

<130>  DK10551EP

<160>  33

<170>  PatentIn version 3.3

<210>  1
<211>  7906
<212>  DNA
<213>  Human papillomavirus type 16

<400>  1
actacaataa ttcatgtata aaactaaggg cgtaaccgaa atcggttgaa ccgaaaccgg      60

ttagtataaa agcagacatt ttatgcacca aaagagaact gcaatgtttc aggacccaca     120

ggagcgaccc agaaagttac cacagttatg cacagagctg caaacaacta tacatgatat     180

aatattagaa tgtgtgtact gcaagcaaca gttactgcga cgtgaggtat atgactttgc     240

ttttcgggat ttatgcatag tatatagaga tgggaatcca tatgctgtat gtgataaatg     300

tttaaagttt tattctaaaa ttagtgagta tagacattat tgttatagtt tgtatggaac     360

aacattagaa cagcaataca acaaaccgtt gtgtgatttg ttaattaggt gtattaactg     420

tcaaaagcca ctgtgtcctg aagaaaagca aagacatctg gacaaaaagc aaagattcca     480

taatataagg ggtcggtgga ccggtcgatg tatgtcttgt tgcagatcat caagaacacg     540

tagagaaacc cagctgtaat catgcatgga gatacaccta cattgcatga atatatgtta     600

gatttgcaac cagagacaac tgatctctac tgttatgagc aattaaatga cagctcagag     660

gaggaggatg aaatagatgg tccagctgga caagcagaac cggacagagc ccattacaat     720

attgtaacct tttgttgcaa gtgtgactct acgcttcggt tgtgcgtaca aagcacacac     780

gtagacattc gtactttgga agacctgtta atgggcacac taggaattgt gtgccccatc     840

tgttctcaga aaccataatc taccatggct gatcctgcag gtaccaatgg ggaagagggt     900

acgggatgta atggatggtt ttatgtagag gctgtagtgg aaaaaaaaac aggggatgct     960

atatcagatg acgagaacga aaatgacagt gatacaggtg aagatttggt agattttata    1020

gtaaatgata atgattattt aacacaggca gaaacagaga cagcacatgc gttgtttact    1080

gcacaggaag caaaacaaca tagagatgca gtacaggttc taaaacgaaa gtatttgggt    1140

agtccactta gtgatattag tggatgtgta gacaataata ttagtcctag attaaaagct    1200

atatgtatag aaaaacaaag tagagctgca aaaaggagat tatttgaaag cgaagacagc    1260

gggtatggca atactgaagt ggaaactcag cagatgttac aggtagaagg gcgccatgag    1320

actgaaacac catgtagtca gtatagtggt ggaagtgggg gtggttgcag tcagtacagt    1380

agtggaagtg ggggagaggg tgttagtgaa agacacacta tatgccaaac accacttaca    1440

aatattttaa atgtactaaa aactagtaat gcaaaggcag caatgttagc aaaatttaaa    1500

gagttatacg gggtgagttt ttcagaatta gtaagaccat ttaaaagtaa taaatcaacg 1560
tgttgcgatt ggtgtattgc tgcatttgga cttacaccca gtatagctga cagtataaaa 1620
acactattac aacaatattg tttatattta cacattcaaa gtttagcatg ttcatgggga 1680
atggttgtgt tactattagt aagatataaa tgtggaaaaa atagagaaac aattgaaaaa 1740
ttgctgtcta aactattatg tgtgtctcca atgtgtatga tgatagagcc tccaaaattg 1800
cgtagtacag cagcagcatt atattggtat aaaacaggta tatcaaatat tagtgaagtg 1860
tatggagaca cgccagaatg gatacaaaga caaacagtat tacaacatag ttttaatgat 1920
tgtacatttg aattatcaca gatggtacaa tgggcctacg ataatgacat agtagacgat 1980
agtgaaattg catataaata tgcacaattg gcagacacta atagtaatgc aagtgccttt 2040
ctaaaaagta attcacaggc aaaaattgta aaggattgtg caacaatgtg tagacattat 2100
aaacgagcag aaaaaaaaca aatgagtatg agtcaatgga taaaatatag atgtgatagg 2160
gtagatgatg gaggtgattg gaagcaaatt gttatgtttt taaggtatca aggtgtagag 2220
tttatgtcat ttttaactgc attaaaaaga tttttgcaag gcatacctaa aaaaaattgc 2280
atattactat atggtgcagc taacacaggt aaatcattat ttggtatgag tttaatgaaa 2340
tttctgcaag ggtctgtaat atgttttgta aattctaaaa gccatttttg gttacaacca 2400
ttagcagatg ccaaaatagg tatgttagat gatgctacag tgccctgttg gaactacata 2460
gatgacaatt taagaaatgc attggatgga aatttagttt ctatggatgt aaagcataga 2520
ccattggtac aactaaaatg ccctccatta ttaattacat ctaacattaa tgctggtaca 2580
gattctaggt ggccttattt acataataga ttggtggtgt ttacatttcc taatgagttt 2640
ccatttgacg aaaacggaaa tccagtgtat gagcttaatg ataagaactg gaaatccttt 2700
ttctcaagga cgtggtccag attaagtttg cacgaggacg aggacaagga aaacgatgga 2760
gactctttgc caacgtttaa atgtgtgtca ggacaaaata ctaacacatt atgaaaatga 2820
tagtacagac ctacgtgacc atatagacta ttggaaacac atgcgcctag aatgtgctat 2880
ttattacaag gccagagaaa tgggatttaa acatattaac caccaagtgg tgccaacact 2940
ggctgtatca aagaataaag cattacaagc aattgaactg caactaacgt tagaaacaat 3000
atataactca caatatagta atgaaaagtg gacattacaa gacgttagcc ttgaagtgta 3060
tttaactgca ccaacaggat gtataaaaaa acatggatat acagtggaag tgcagtttga 3120
tggagacata tgcaatacaa tgcattatac aaactggaca catatatata tttgtgaaga 3180
agcatcagta actgtggtag agggtcaagt tgactattat ggtttatatt atgttcatga 3240
aggaatacga acatattttg tgcagtttaa agatgatgca gaaaaatata gtaaaaataa 3300
agtatgggaa gttcatgcgg gtggtcaggt aatattatgt cctacatctg tgtttagcag 3360
caacgaagta tcctctcctg aaattattag gcagcacttg gccaaccacc ccgccgcgac 3420
ccataccaaa gccgtcgcct tgggcaccga agaaacacag acgactatcc agcgaccaag 3480
atcagagcca gacaccggaa acccctgcca caccactaag ttgttgcaca gagactcagt 3540
ggacagtgct ccaatcctca ctgcatttaa cagctcacac aaaggacgga ttaactgtaa 3600

tagtaacact acacccatag tacatttaaa aggtgatgct aatactttaa aatgtttaag 3660
atatagattt aaaaagcatt gtacattgta tactgcagtg tcgtctacat ggcattggac 3720
aggacataat gtaaaacata aaagtgcaat tgttacactt acatatgata gtgaatggca 3780
acgtgaccaa tttttgtctc aagttaaaat accaaaaact attacagtgt ctactggatt 3840
tatgtctata tgacaaatct tgatactgca tccacaacat tactggcgtg ctttttgctt 3900
tgcttttgtg tgcttttgtg tgtctgccta ttaatacgtc cgctgctttt gtctgtgtct 3960
acatacacat cattaataat attggtatta ctattgtgga taacagcagc ctctgcgttt 4020
aggtgtttta ttgtatatat tatatttgtt tatataccat tatttttaat acatacacat 4080
gcacgctttt taattacata atgtatatgt acataatgta attgttacat ataattgttg 4140
tataccataa cttactattt tttctttttt attttcatat ataatttttt tttttgtttg 4200
tttgtttgtt ttttaataaa ctgttattac ttaacaatgc gacacaaacg ttctgcaaaa 4260
cgcacaaaac gtgcatcggc tacccaactt tataaaacat gcaaacaggc aggtacatgt 4320
ccacctgaca ttatacctaa ggttgaaggc aaaactattg ctgatcaaat attacaatat 4380
ggaagtatgg gtgtattttt tggtgggtta ggaattggaa cagggtcggg tacaggcgga 4440
cgcactgggt atattccatt gggaacaagg cctcccacag ctacagatac acttgctcct 4500
gtaagacccc ctttaacagt agatcctgtg ggcccttctg atccttctat agtttcttta 4560
gtggaagaaa ctagttttat tgatgctggt gcaccaacat ctgtaccttc cattccccca 4620
gatgtatcag gatttagtat tactacttca actgatacca cacctgctat attagatatt 4680
aataatactg ttactactgt tactacacat aataatccca ctttcactga cccatctgta 4740
ttgcagcctc caacacctgc agaaactgga gggcatttta cactttcatc atccactatt 4800
agtacacata attatgaaga aattcctatg gatacattta ttgttagcac aaaccctaac 4860
acagtaacta gtagcacacc cataccaggg tctcgcccag tggcacgcct aggattatat 4920
agtcgcacaa cacaacaggt taaagttgta gaccctgctt ttgtaaccac tcccactaaa 4980
cttattacat atgataatcc tgcatatgaa ggtatagatg tggataatac attatatttt 5040
tctagtaatg ataatagtat taatatagct ccagatcctg actttttgga tatagttgct 5100
ttacataggc cagcattaac ctctaggcgt actggcatta ggtacagtag aattggtaat 5160
aaacaaacac tacgtactcg tagtggaaaa tctataggtg ctaaggtaca ttattattat 5220
gatttaagta ctattgatcc tgcagaagaa atagaattac aaactataac accttctaca 5280
tatactacca cttcacatgc agcctcacct acttctatta ataatggatt atatgatatt 5340
tatgcagatg actttattac agatacttct acaaccccgg taccatctgt accctctaca 5400
tctttatcag gttatattcc tgcaaataca acaattcctt ttggtggtgc atacaatatt 5460
cctttagtat caggtcctga tatacccatt aatataactg accaagctcc ttcattaatt 5520
cctatagttc cagggtctcc acaatataca attattgctg atgcaggtga cttttattta 5580
catcctagtt attacatgtt acgaaaacga cgtaaacgtt taccatattt tttttcagat 5640

gtctctttgg ctgcctagtg aggccactgt ctacttgcct cctgtcccag tatctaaggt 5700
tgtaagcacg gatgaatatg ttgcacgcac aaacatatat tatcatgcag gaacatccag 5760
actacttgca gttggacatc cctattttcc tattaaaaaa cctaacaata acaaaatatt 5820
agttcctaaa gtatcaggat tacaatacag ggtatttaga atacatttac ctgaccccaa 5880
taagtttggt tttcctgaca cctcatttta taatccagat acacagcggc tggtttgggc 5940
ctgtgtaggt gttgaggtag gtcgtggtca gccattaggt gtgggcatta gtggccatcc 6000
tttattaaat aaattggatg acacagaaaa tgctagtgct tatgcagcaa atgcaggtgt 6060
ggataataga gaatgtatat ctatggatta caaacaaaca caattgtgtt taattggttg 6120
caaaccacct ataggggaac actggggcaa aggatcccca tgtaccaatg ttgcagtaaa 6180
tccaggtgat tgtccaccat tagagttaat aaacacagtt attcaggatg gtgatatggt 6240
tgatactggc tttggtgcta tggactttac tacattacag gctaacaaaa gtgaagttcc 6300
actggatatt tgtacatcta tttgcaaata tccagattat attaaaatgg tgtcagaacc 6360
atatggcgac agcttatttt tttatttacg aagggaacaa atgtttgtta gacatttatt 6420
taatagggct ggtactgttg gtgaaaatgt accagacgat ttatacatta aaggctctgg 6480
gtctactgca aatttagcca gttcaaatta ttttcctaca cctagtggtt ctatggttac 6540
ctctgatgcc caaatattca ataaacctta ttggttacaa cgagcacagg gccacaataa 6600
tggcatttgt tggggtaacc aactatttgt tactgttgtt gatactacac gcagtacaaa 6660
tatgtcatta tgtgctgcca tatctacttc agaaactaca tataaaaata ctaactttaa 6720
ggagtaccta cgacatgggg aggaatatga tttacagttt atttttcaac tgtgcaaaat 6780
aaccttaact gcagacgtta tgacatacat acattctatg aattccacta ttttggagga 6840
ctggaatttt ggtctacaac ctcccccagg aggcacacta gaagatactt ataggtttgt 6900
aacatcccag gcaattgctt gtcaaaaaca tacacctcca gcacctaaag aagatcccct 6960
taaaaaatac actttttggg aagtaaattt aaaggaaaag ttttctgcag acctagatca 7020
gtttccttta ggacgcaaat ttttactaca agcaggattg aaggccaaac caaaatttac 7080
attaggaaaa cgaaaagcta cacccaccac ctcatctacc tctacaactg ctaaacgcaa 7140
aaaacgtaag ctgtaagtat tgtatgtatg ttgaattagt gttgtttgtt gtgtatatgt 7200
ttgtatgtgc ttgtatgtgc ttgtaaatat taagttgtat gtgtgtttgt atgtatggta 7260
taataaacac gtgtgtatgt gtttttaaat gcttgtgtaa ctattgtgtc atgcaacata 7320
aataaactta ttgtttcaac acctactaat tgtgttgtgg ttattcattg tatataaact 7380
atatttgcta catcctgttt ttgttttata tatactatat tttgtagcgc cagcggccat 7440
tttgtagctt caaccgaatt cggttgcatg ctttttggca caaaatgtgt ttttttaaat 7500
agttctatgt cagcaactat ggtttaaact tgtacgtttc ctgcttgcca tgcgtgccaa 7560
atccctgttt tcctgacctg cactgcttgc caaccattcc attgtttttt acactgcact 7620
atgtgcaact actgaatcac tatgtacatt gtgtcatata aaataaatca ctatgcgcca 7680
acgccttaca taccgctgtt aggcacatat ttttggcttg ttttaactaa cctaattgca 7740

tatttggcat aaggtttaaa cttctaaggc caactaaatg tcaccctagt tcatacatga 7800

actgtgtaaa ggttagtcat acattgttca tttgtaaaac tgcacatggg tgtgtgcaaa 7860

ccgttttggg ttacacattt acaagcaact tatataataa tactaa 7906

<210> 2
<211> 50
<212> PRT
<213> Human papillomavirus type 16

<400> 2

Met His Gln Lys Arg Thr Ala Met Phe Gln Asp Pro Gln Glu Arg Pro
1               5                   10                  15

Arg Lys Leu Pro Gln Leu Cys Thr Glu Leu Gln Thr Thr Ile His Asp
            20                  25                  30

Ile Ile Leu Glu Cys Val Tyr Cys Lys Gln Gln Leu Leu Arg Arg Glu
        35                  40                  45

Val Tyr
    50

<210> 3
<211> 153
<212> DNA
<213> Human papillomavirus type 16

<400> 3
atgcaccaaa agagaactgc aatgtttcag gacccacagg agcgacccag aaagttacca 60

cagttatgca cagagctgca aacaactata catgatataa tattagaatg tgtgtactgc 120

aagcaacagt tactgcgacg tgaggtgtat taa 153

<210> 4
<211> 12
<212> DNA
<213> Human papillomavirus type 16

<400> 4
cgtgaggtgt at 12

<210> 5
<211> 7
<212> PRT
<213> Human papillomavirus type 16

<400> 5

Leu Leu Arg Arg Glu Val Tyr
1               5

<210> 6
<211> 53
<212> PRT
<213> Human papillomavirus type 16

<400>  6

Met His Gln Lys Arg Thr Ala Met Phe Gln Asp Pro Gln Glu Arg Pro
1               5                   10                  15

Arg Lys Leu Pro Gln Leu Cys Thr Glu Leu Gln Thr Thr Ile His Asp
            20                  25                  30

Ile Ile Leu Glu Cys Val Tyr Cys Lys Gln Gln Leu Leu Arg Arg Glu
        35                  40                  45

Ile Ile Lys Asn Thr
    50

<210>  7
<211>  178
<212>  DNA
<213>  Human papillomavirus type 16

<400>  7
atgcaccaaa agagaactgc aatgtttcag gacccacagg agcgacccag aaagttacca      60

cagttatgca cagagctgca aacaactata catgatataa tattagaatg tgtgtactgc     120

aagcaacagt tactgcgacg tgagatcatc aagaacacgt agagaaaccc agctgtaa       178

<210>  8
<211>  92
<212>  PRT
<213>  Human papillomavirus type 16

<400>  8

Met Ala Asp Pro Ala Ala Ala Thr Lys Tyr Pro Leu Leu Lys Leu Leu
1               5                   10                  15

Gly Ser Thr Trp Pro Thr Thr Pro Pro Arg Pro Ile Pro Lys Pro Ser
            20                  25                  30

Pro Trp Ala Pro Lys Lys His Arg Arg Leu Ser Ser Asp Gln Asp Gln
        35                  40                  45

Ser Gln Thr Pro Glu Thr Pro Ala Thr Pro Leu Ser Cys Cys Thr Glu
    50                  55                  60

Thr Gln Trp Thr Val Leu Gln Ser Ser Leu His Leu Thr Ala His Thr
65                  70                  75                  80

Lys Asp Gly Leu Thr Val Ile Val Thr Leu His Pro
                85                  90

<210>  9
<211>  279
<212>  DNA
<213>  Human papillomavirus type 16

<400>  9
atggctgatc ctgcagcagc aacgaagtat cctctcctga aattattagg cagcacttgg      60

ccaaccaccc cgccgcgacc cataccaaag ccgtcgcctt gggcaccgaa gaaacacaga 120

cgactatcca gcgaccaaga tcagagccag acaccggaaa cccctgccac accactaagt 180

tgttgcacag agactcagtg gacagtgctc caatcctcac tgcatttaac agctcacaca 240

aaggacggat taactgtaat agtaacacta cacccatag 279

<210> 10
<211> 82
<212> PRT
<213> Human papillomavirus type 16

<400> 10

Met Ala Asp Pro Ala Asp Ser Arg Trp Pro Tyr Leu His Asn Arg Leu
1               5                   10                  15

Val Val Phe Thr Phe Pro Asn Glu Phe Pro Phe Asp Glu Asn Gly Asn
            20                  25                  30

Pro Val Tyr Glu Leu Asn Asp Lys Asn Trp Lys Ser Phe Phe Ser Arg
        35                  40                  45

Thr Trp Ser Arg Leu Ser Leu His Glu Asp Glu Asp Lys Glu Asn Asp
    50                  55                  60

Gly Asp Ser Leu Pro Thr Phe Lys Cys Val Ser Gly Gln Asn Thr Asn
65                  70                  75                  80

Thr Leu

<210> 11
<211> 249
<212> DNA
<213> Human papillomavirus type 16

<400> 11
atggctgatc ctgcagattc yaggtggcct tatttacata atagattggt ggtgtttaca 60

tttcctaatg agtttccatt tgacgaaaac ggaaatccag tgtatgagct taatgataag 120

aactggaaat cctttttctc aaggacgtgg tccagattaa gtttgcacga ggacgaggac 180

aaggaaaacg atggagactc tttgccaacg tttaaatgtg tgtcaggaca aaatactaac 240

acattatga 249

<210> 12
<211> 12
<212> DNA
<213> Human papillomavirus type 16

<400> 12
ctgcagattc ya 12

<210> 13
<211> 7

<212>  PRT
<213>  Human papillomavirus type 16

<400>  13

Asp Pro Ala Asp Ser Arg Trp
1               5


<210>  14
<211>  12
<212>  DNA
<213>  Human papillomavirus type 16

<400>  14
ctrcagcagc ra                                                          12


<210>  15
<211>  12
<212>  DNA
<213>  Human papillomavirus type 16

<400>  15
cgtgaggtgt at                                                          12


<210>  16
<211>  7
<212>  PRT
<213>  Human papillomavirus type 16

<400>  16

Asp Pro Ala Ala Ala Thr Lys
1               5


<210>  17
<211>  24
<212>  DNA
<213>  Human papillomavirus type 16

<400>  17
ggatttccgt tttcgtcaaa tgga                                             24


<210>  18
<211>  21
<212>  DNA
<213>  Human papillomavirus type 16

<400>  18
catctgttct cagaaaccat a                                                21


<210>  19
<211>  20
<212>  DNA
<213>  Human papillomavirus type 16

<400>  19
ctgtgtttct tyggtgccca                                                  20


<210>  20
<211>  24
<212>  DNA
<213>  Human papillomavirus type 16

<400>  20
acaagacata catcgaccgg tcca                                              24


<210>  21
<211>  21
<212>  DNA
<213>  Human papillomavirus type 16

<400>  21
gtgtactgca agcaacagtt a                                                 21


<210>  22
<211>  21
<212>  DNA
<213>  Human papillomavirus type 16

<400>  22
gatcagttgt ctctggttgc a                                                 21


<210>  23
<211>  7
<212>  PRT
<213>  Human papillomavirus type 16

<400>  23

Asp Pro Ala Asp Ser Arg Trp
1               5


<210>  24
<211>  7
<212>  PRT
<213>  Human papillomavirus type 16

<400>  24

Leu Leu Arg Arg Glu Val Tyr
1               5


<210>  25
<211>  7
<212>  PRT
<213>  Human papillomavirus type 16

<400>  25

Asp Pro Ala Ala Ala Thr Lys
1               5


<210>  26
<211>  6
<212>  PRT
<213>  Human papillomavirus type 16

<400>  26

Arg Arg Glu Ile Ile Lys
1               5


<210>  27
<211>  505

<212>  PRT
<213>  Human papillomavirus type 16

<400>  27

Met Ser Leu Trp Leu Pro Ser Glu Ala Thr Val Tyr Leu Pro Pro Val
1               5                   10                  15

Pro Val Ser Lys Val Val Ser Thr Asp Glu Tyr Val Ala Arg Thr Asn
            20                  25                  30

Ile Tyr Tyr His Ala Gly Thr Ser Arg Leu Leu Ala Val Gly His Pro
        35                  40                  45

Tyr Phe Pro Ile Lys Lys Pro Asn Asn Asn Lys Ile Leu Val Pro Lys
    50                  55                  60

Val Ser Gly Leu Gln Tyr Arg Val Phe Arg Ile His Leu Pro Asp Pro
65                  70                  75                  80

Asn Lys Phe Gly Phe Pro Asp Thr Ser Phe Tyr Asn Pro Asp Thr Gln
                85                  90                  95

Arg Leu Val Trp Ala Cys Val Gly Val Glu Val Gly Arg Gly Gln Pro
            100                 105                 110

Leu Gly Val Gly Ile Ser Gly His Pro Leu Leu Asn Lys Leu Asp Asp
        115                 120                 125

Thr Glu Asn Ala Ser Ala Tyr Ala Ala Asn Ala Gly Val Asp Asn Arg
    130                 135                 140

Glu Cys Ile Ser Met Asp Tyr Lys Gln Thr Gln Leu Cys Leu Ile Gly
145                 150                 155                 160

Cys Lys Pro Pro Ile Gly Glu His Trp Gly Lys Gly Ser Pro Cys Thr
                165                 170                 175

Asn Val Ala Val Asn Pro Gly Asp Cys Pro Pro Leu Glu Leu Ile Asn
            180                 185                 190

Thr Val Ile Gln Asp Gly Asp Met Val Asp Thr Gly Phe Gly Ala Met
        195                 200                 205

Asp Phe Thr Thr Leu Gln Ala Asn Lys Ser Glu Val Pro Leu Asp Ile
    210                 215                 220

Cys Thr Ser Ile Cys Lys Tyr Pro Asp Tyr Ile Lys Met Val Ser Glu
225                 230                 235                 240

Pro Tyr Gly Asp Ser Leu Phe Phe Tyr Leu Arg Arg Glu Gln Met Phe
                245                 250                 255

Val Arg His Leu Phe Asn Arg Ala Gly Thr Val Gly Glu Asn Val Pro
            260                 265                 270

Asp Asp Leu Tyr Ile Lys Gly Ser Gly Ser Thr Ala Asn Leu Ala Ser
        275                 280                 285

Ser Asn Tyr Phe Pro Thr Pro Ser Gly Ser Met Val Thr Ser Asp Ala
    290                 295                 300

Gln Ile Phe Asn Lys Pro Tyr Trp Leu Gln Arg Ala Gln Gly His Asn
305                 310                 315                 320

Asn Gly Ile Cys Trp Gly Asn Gln Leu Phe Val Thr Val Val Asp Thr
                325                 330                 335

Thr Arg Ser Thr Asn Met Ser Leu Cys Ala Ala Ile Ser Thr Ser Glu
            340                 345                 350

Thr Thr Tyr Lys Asn Thr Asn Phe Lys Glu Tyr Leu Arg His Gly Glu
        355                 360                 365

Glu Tyr Asp Leu Gln Phe Ile Phe Gln Leu Cys Lys Ile Thr Leu Thr
    370                 375                 380

Ala Asp Val Met Thr Tyr Ile His Ser Met Asn Ser Thr Ile Leu Glu
385                 390                 395                 400

Asp Trp Asn Phe Gly Leu Gln Pro Pro Pro Gly Gly Thr Leu Glu Asp
                405                 410                 415

Thr Tyr Arg Phe Val Thr Ser Gln Ala Ile Ala Cys Gln Lys His Thr
            420                 425                 430

Pro Pro Ala Pro Lys Glu Asp Pro Leu Lys Lys Tyr Thr Phe Trp Glu
        435                 440                 445

Val Asn Leu Lys Glu Lys Phe Ser Ala Asp Leu Asp Gln Phe Pro Leu
    450                 455                 460

Gly Arg Lys Phe Leu Leu Gln Ala Gly Leu Lys Ala Lys Pro Lys Phe
465                 470                 475                 480

Thr Leu Gly Lys Arg Lys Ala Thr Pro Thr Thr Ser Ser Thr Ser Thr
                485                 490                 495

Thr Ala Lys Arg Lys Lys Arg Lys Leu
            500                 505

<210>  28
<211>  1518
<212>  DNA
<213>  Human papillomavirus type 16

<400>  28
atgtctcttt ggctgcctag tgaggccact gtctacttgc ctcctgtccc agtatctaag      60

gttgtaagca cggatgaata tgttgcacgc acaaacatat attatcatgc aggaacatcc     120

agactacttg cagttggaca tccctatttt cctattaaaa aacctaacaa taacaaaata     180

ttagttccta aagtatcagg attacaatac agggtattta gaatacattt acctgacccc     240

aataagtttg gttttcctga cacctcattt tataatccag atacacagcg gctggtttgg     300

gcctgtgtag gtgttgaggt aggtcgtggt cagccattag gtgtgggcat tagtggccat     360

cctttattaa ataaattgga tgacacagaa aatgctagtg cttatgcagc aaatgcaggt     420

gtggataata gagaatgtat atctatggat tacaaacaaa cacaattgtg tttaattggt     480

tgcaaaccac ctatagggga acactggggc aaaggatccc catgtaccaa tgttgcagta     540

aatccaggtg attgtccacc attagagtta ataaacacag ttattcagga tggtgatatg     600

gttgatactg gctttggtgc tatggacttt actacattac aggctaacaa aagtgaagtt     660

ccactggata tttgtacatc tatttgcaaa tatccagatt atattaaaat ggtgtcagaa     720

ccatatggcg acagcttatt tttttattta cgaagggaac aaatgtttgt tagacattta     780

tttaataggg ctggtactgt tggtgaaaat gtaccagacg atttatacat taaaggctct     840

gggtctactg caaatttagc cagttcaaat tattttccta cacctagtgg ttctatggtt     900

acctctgatg cccaaatatt caataaacct tattggttac aacgagcaca gggccacaat     960

aatggcattt gttggggtaa ccaactattt gttactgttg ttgatactac acgcagtaca    1020

aatatgtcat tatgtgctgc catatctact tcagaaacta catataaaaa tactaacttt    1080

aaggagtacc tacgacatgg ggaggaatat gatttacagt ttatttttca actgtgcaaa    1140

ataaccttaa ctgcagacgt tatgacatac atacattcta tgaattccac tattttggag    1200

gactggaatt ttggtctaca acctccccca ggaggcacac tagaagatac ttataggttt    1260

gtaacatccc aggcaattgc ttgtcaaaaa catacacctc cagcacctaa agaagatccc    1320

cttaaaaaat acactttttg ggaagtaaat ttaaaggaaa agttttctgc agacctagat    1380

cagtttcctt taggacgcaa atttttacta caagcaggat tgaaggccaa accaaaattt    1440

acattaggaa aacgaaaagc tacacccacc acctcatcta cctctacaac tgctaaacgc    1500

aaaaaacgta agctgtaa                                                  1518


<210>  29
<211>  12
<212>  DNA
<213>  Human papillomavirus type 16

<400>  29
taaaagatgt ct                                                          12


<210>  30
<211>  477
<212>  DNA
<213>  Human papillomavirus type 16

<400>  30

atgcaccaaa agagaactgc aatgtttcag gacccacagg agcgacccag aaagttacca      60

cagttatgca cagagctgca aacaactata catgatataa tattagaatg tgtgtactgc     120

aagcaacagt tactgcgacg tgaggtatat gactttgctt ttcgggattt atgcatagta     180

tatagagatg ggaatccata tgctgtatgt gataaatgtt taaagtttta ttctaaaatt     240

agtgagtata gacattattg ttatagtttg tatggaacaa cattagaaca gcaatacaac     300

aaaccgttgt gtgatttgtt aattaggtgt attaactgtc aaaagccact gtgtcctgaa     360

gaaaagcaaa gacatctgga caaaaagcaa agattccata atataagggg tcggtggacc     420

ggtcgatgta tgtcttgttg cagatcatca agaacacgta gagaaaccca gctgtaa        477


<210>  31
<211>  297
<212>  DNA
<213>  Human papillomavirus type 16

<400>  31
atgcatggag atacacctac attgcatgaa tatatgttag atttgcaacc agagacaact      60

gatctctact gttatgagca attaaatgac agctcagagg aggaggatga aatagatggt     120

ccagctggac aagcagaacc ggacagagcc cattacaata ttgtaacctt ttgttgcaag     180

tgtgactcta cgcttcggtt gtgcgtacaa agcacacacg tagacattcg tactttggaa     240

gacctgttaa tgggcacact aggaattgtg tgccccatct gttctcagaa accataa        297


<210>  32
<211>  21
<212>  DNA
<213>  Human papillomavirus type 16

<400>  32
aatagtaaca ctacacccat a                                                21


<210>  33
<211>  27
<212>  DNA
<213>  Human papillomavirus type 16

<400>  33
catgataata tatgtttgtg cgtgcaa                                          27